(19) [European Patent Office logo] Europäisches Patentamt European Patent Office Office européen des brevets

(11) **EP 2 416 893 B1**

(12) **EUROPEAN PATENT SPECIFICATION**

(45) Date of publication and mention
of the grant of the patent:
**30.10.2013 Bulletin 2013/44**

(51) Int Cl.:
*B05D 5/12* (2006.01)          *G01N 27/26* (2006.01)
*G01N 27/49* (2006.01)          *C12Q 1/00* (2006.01)
*G01N 33/543* (2006.01)

(21) Application number: **10746715.1**

(22) Date of filing: **23.02.2010**

(86) International application number:
**PCT/US2010/025090**

(87) International publication number:
**WO 2010/099122 (02.09.2010 Gazette 2010/35)**

(54) **MICROSECOND RESPONSE ELECTROCHEMICAL SENSORS AND METHODS THEREOF**

ELEKTROCHEMISCHE SENSOREN MIT MIKROSEKUNDENANTWORT UND VERFAHREN DAFÜR

CAPTEURS ÉLECTROCHIMIQUES À RÉPONSE EN MICROSECONDES ET LEURS PROCÉDÉS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **24.02.2009 US 208419 P**

(43) Date of publication of application:
**15.02.2012 Bulletin 2012/07**

(73) Proprietor: **Ultradian Diagnostics LLC
Rensselaer, NY 12144-3425 (US)**

(72) Inventor: **WILLIS, John, P.
Buskirk
NY 12028 (US)**

(74) Representative: **Gervasi, Gemma et al
Notarbartolo & Gervasi S.p.A.
Corso di Porta Vittoria 9
20122 Milano (IT)**

(56) References cited:
**US-A1- 2006 076 236      US-A1- 2007 299 617**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD**

This technology generally relates to electrochemical sensors and, more particularly, to a system including microsecond electrochemical sensors and cell for the measurement of analytes and methods thereof.

**BACKGROUND**

Prior art sensor systems; in particular, minimally invasive, enzymatic, continuous glucose sensors can initiate an inflammatory response when implanted subcutaneously. Because the sensor is a foreign object, the body's immune system attempts to dissolve or eject the sensor from the body. If this is not possible, the sensor is encapsulated within an avascular fibrin capsule that reduces the analyte supply to the implanted sensor. Because of this, less mass of analyte can diffuse across the diffusion limiting barrier into the active zone so the response and sensitivity of the sensor decreases. This can lead to significant inaccuracy in the measurement of analytes such as glucose. The only way to correct for this loss in sensitivity is to perform a re-calibration of the in vivo sensor.

Prior art amperometric electrochemical sensors or cells rely on a calibrated current output response from a working electrode to measure analyte concentration. These measurements can be noisy and require minutes to achieve an equilibrium response to an increase in analyte concentration.

What is needed is a rapid, robust measurement of analyte concentration.

SUMMARY

A system for the measurement of analyte concentration that includes an electrochemical cell having a working electrode coated with a protein layer and a diffusion limiting barrier covering the protein layer, and a counter electrode; a voltage source which provides a voltage between the working electrode and the counter electrode when electrically connected by a conductive medium; and a computing system which measures the dynamic voltage output to the counter electrode within a time period prior to a response from the working electrode is shown.

An electronic circuit for measuring voltage or charge of a counter electrode within a time period prior to a working electrode response including a voltage waveform source between a counter and working electrode and an analog to digital converter capable of sampling the voltages at a rate sufficient to yield two or more voltage measurements within a time period between 0 and a time point defined by $tV_{max}$ is also shown.

A method for determining a concentration of at least one analyte, the method includes: providing an electrochemical cell including a working electrode coated with a protein layer and a diffusion limiting barrier covering the protein layer, and a counter electrode; a voltage source which provides a voltage between the working electrode and the counter electrode; and a computing system which measures the dynamic voltage output to the counter electrode; positioning the working electrode and the counter electrode in an electrically conductive medium; applying voltage from the voltage source to the working electrode and the counter electrode; detecting the applied voltage to the counter electrode prior to the working electrode response; and determining a concentration of at least one analyte based on the rate of change of the voltage supplied to the counter electrode or a single voltage measurement or an average of voltage measurements or other functions related to the counter electrode voltage.

**BRIEF DESCRIPTION OF THE DRAWINGS**

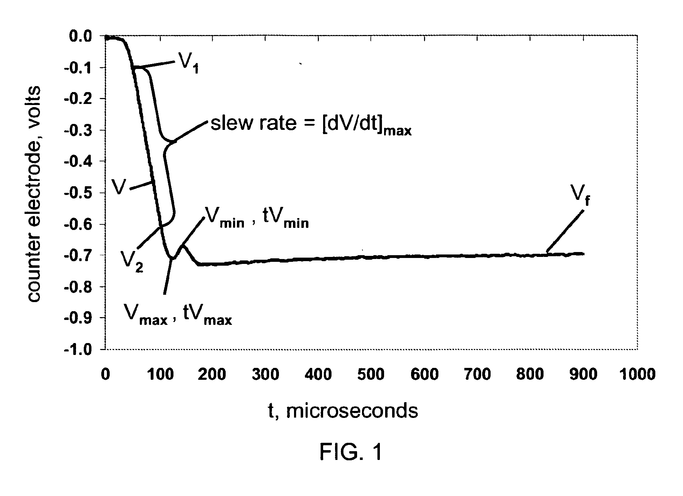

FIG. 1 shows a graph of the voltage output of an op amp driving a counter electrode versus time. A number of parameters proportional to analyte concentration can be identified such as $V_1$ & $V_2$, two voltage points between which the slope of the counter voltage can be used to determine the slew rate response (dV/dt, V/$\mu$sec), the maximum counter electrode voltage ($V_{max}$), the minimum voltage ($V_{min}$), the final or steady state counter electrode voltage ($V_f$), and also the time points corresponding to the voltages, $V_{max}$ and $V_{min}$, defined as $tV_{max}$ and $tV_{min}$, respectively and all the functions of the counter electrode voltage are proportional to analyte concentration;

FIG. 2 shows a three-electrode electrochemical cell with electrodes held within a conductive medium;

FIG. 3 illustrates an example of an electrochemical cell wherein a working electrode (W) is coated with a protein layer, such as glucose oxidase (GOx) and gelatin. In addition, the protein layer is covered by a second layer including a diffusion limiting barrier composed of a polymer;.

FIG. 4 is a view of a protein layer, on a working electrode, containing glucose oxidase along with a diffusion limiting barrier on a working electrode versus a counter electrode on the right side of the drawing. The reactions occurring within the active zone of the working electrode also are shown for the reaction of glucose with glucose oxidase;

FIG. 5 is a depiction of a working electrode having a protein layer encapsulated within a diffusion limiting barrier versus a counter electrode. The resistance and capacitance contributions to the impedance between the working electrode surface and the counter electrode surface are shown along with other descriptive terms;

FIG. 6 shows a portion of an electronic circuit for a potentiostat that can be used to apply a voltage between a working electrode and counter electrode of an electrochemical cell. The e-cell (W, R, C) describes where the electrochemical cell (such as in FIGs. 2 and 3) is operationally connected to the electronic circuit;

FIG. 7a shows an example of an in vivo configuration for an implanted three-electrode cell (300a) including working electrode (W), a counter electrode (C), a reference electrode (R), a skin surface (310), a skin thickness (315), subcutaneous tissue and interstitial fluid (320), an active zone (325), a diffusion limiting barrier (330), resistance ($R_s$) between the working and counter electrodes and uncompensated resistance ($R_u$) between the working and reference electrodes;

FIG. 7b shows an example of an in vivo configuration for an implanted three-electrode cell (300b) including a working electrode (W), a counter electrode (C), a reference electrode (R), a skin surface (310), a skin thickness (315), subcutaneous tissue and interstitial fluid (320), an active zone (325), a diffusion limiting barrier (330), biofouling layer (340), resistance ($R_s$) between the working and counter electrodes and uncompensated resistance ($R_u$) between the working and reference electrodes;

FIG. 8a shows an example of an in vivo configuration for a partially implanted three-electrode cell (300c) including an implanted working electrode (W), a counter electrode (350) in electrical contact with a skin surface, an implanted reference electrode (R), a skin surface (310), a skin thickness (315), subcutaneous tissue and interstitial fluid (320), an active zone (325), a diffusion limiting barrier (330), resistance ($R_s$) between the working and counter electrodes and uncompensated resistance ($R_u$) between the working and reference electrodes;

FIG. 8b shows an example of an in vivo configuration for a partially implanted three-electrode cell (300d) including an implanted working electrode (W), a counter electrode (350) in electrical contact with a skin surface, an implanted reference electrode (R), a diffusion limiting barrier (330), a skin surface (310), a skin thickness (315), subcutaneous tissue and interstitial fluid (320), an active zone (325), biofouling layer 340, resistance ($R_s$) between the working and counter electrodes and uncompensated resistance ($R_u$) between the working and reference electrodes;

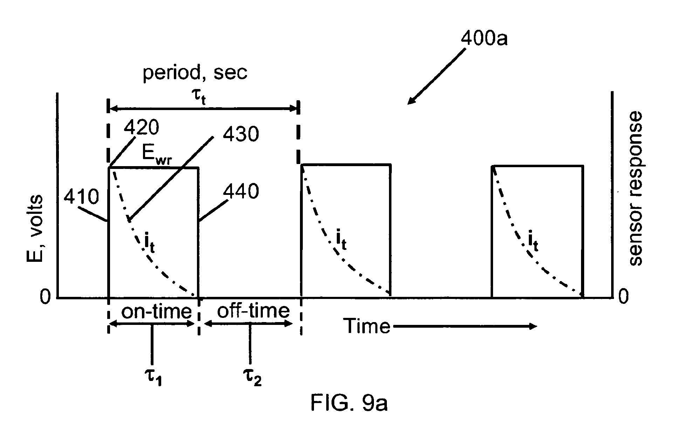

FIG. 9a shows a series (400a) of intermittent square wave voltage pulses, progressing through time, having a total pulse period ($\tau_t$) equal to the sum of a voltage on-time ($\tau_1$) and a voltage off-time. ($\tau_2$). A vertical rectangular box shows a rising voltage or current (410), maximum voltage (420), decaying current vs. time transient ($i_t$) 430, the end of on-time period ($\tau_1$) and falling voltage are defined by (440). The area defined by the time line on the X-axis, vertical solid line (410), horizontal solid line labeled $E_{wr}$ and vertical solid line (440) delineate the square wave voltage pulse, the on-time period ($\tau_1$) and decaying current vs. time transient (430). The line (440) shows a rapid fall-off in the working electrode voltage at the end of the pulse period ($\tau_t$);

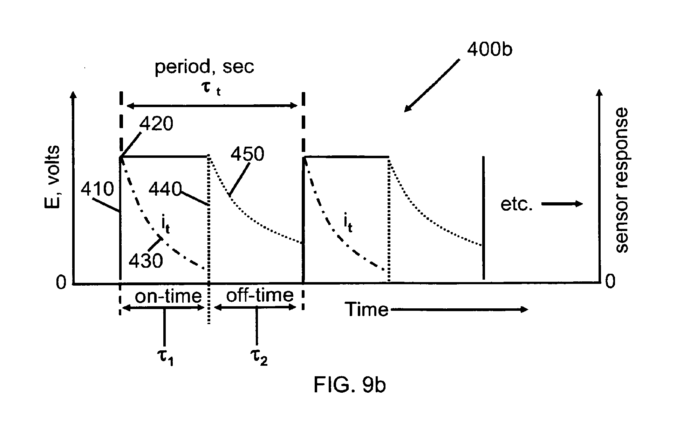

FIG. 9b shows a series (400b) of intermittent square wave voltage pulses, progressing through time, having a total period ($\tau_t$) equal to the sum of a voltage on-time ($\tau_1$) and a voltage off-time. ($\tau_2$). Similar to FIG. 9a, a rectangular box shows a rising voltage or current (410), maximum voltage (420), decaying current vs. time transient ($i_t$) (430), the end of on-time period ($\tau_1$) and falling voltage defined by (440). The area defined by the time line on the X-axis, vertical solid line (410), horizontal solid line labeled as $E_{wr}$ and dotted line (450) describes a decaying working electrode voltage versus time as opposed to the rapid fall (440) in the working electrode voltage in FIG. 9a;

FIG. 10 shows a graph of a linear regression of conductance, $1/R$ ($\Omega^{-1}$ or Siemens), on the Y-axis versus reference glucose concentration on the X-axis. The measurements were obtained with an in vitro electrochemical cell containing a solution of pH 7.4 PBS and a glucose oxidase biosensor as the working electrode;

FIG. 11 illustrates an example of how conductance values in FIG.10 were calculated from current vs. time transients by plotting the natural log (Ln $i_w$) of the working electrode current on the Y-axis vs. transient time (t, microseconds) on the X-axis. A plot of the natural log (Ln) of the working electrode current versus the transient time has a slope of $-1/RC$, and an intercept at zero glucose concentration of Ln $[i_W]_o$ which is also equal to Ln [E/R]. The anti-log of the zero intercept represents the offset current;

FIG. 12 shows a graph of counter electrode voltage (gray trace) and working electrode current response (black trace) of an in vitro electrochemical cell (platinum counter electrode, glucose oxidase working electrode and Ag/AgCl reference electrode, (e.g. cell in FIG. 3)), to a single application of a voltage pulse applied between the counter and working electrodes. Note that the working electrode response is delayed by about 200 microseconds or until the counter electrode voltage reaches a plateau;

FIG. 13 shows an example of the measurement of in vitro working electrode response time (1.25 min) derived from the addition of glucose to an electrochemical cell having an amperometric glucose oxidase working electrode in pH 7.4 PBS;

FIG. 14 shows discrete, stepped responses from the serial addition of glucose to an in vitro electrochemical cell having an amperometric glucose oxidase working electrode in pH 7.4 PBS;

FIG. 15 illustrates the in vivo response of an intradermal, amperometric glucose oxidase biosensor, implanted within the skin of a swine, to a bolus injection of glucose at approximately 180 minutes. The working electrode response exhibits a continuous trace between approximately 180 minutes and 280 minutes without the discrete steps shown in FIG. 14;

FIG. 16 shows op amp output voltages to a counter electrode in response to varying concentrations of glucose added to an in vitro electrochemical cell having a platinum counter electrode, Ag/AgCl reference electrode and a working electrode composed of an amperometric glucose oxidase sensor in pH 7.4 PBS. Glucose concentrations are assigned to each counter voltage response;

FIG. 17a shows a graph of the slopes, obtained from linear regression of the output voltage to a counter electrode vs. time on the Y-axis, versus reference glucose concentration on the X-axis (e.g. data in FIG.16). The slopes and intercepts were measured versus glucose concentration from the decreasing counter voltages, in FIG. 16, between approximately 50 $\mu$sec to about 120 $\mu$sec, the time range depends on the glucose concentration;

FIG. 17b shows a linear regression correlation plot of measured glucose concentrations on the Y-axis, obtained from the slope and intercept data in FIG. 17a, versus reference glucose concentration on the X-axis;

FIG. 18a shows a graph of the intercepts on the Y-axis, obtained from linear regression of the output voltage to a counter electrode vs. time (see FIG. 16), versus glucose concentration on the X-axis;

FIG. 18b shows a linear regression correlation plot of measured glucose concentrations on the Y-axis, obtained from the slope and intercept data shown in FIG 18a, versus reference glucose concentration on the X-axis;

FIG. 19 shows a linear regression graph of $V_{max}$ (see FIG. 1) volts on the Y-axis versus reference glucose concentration on the X-axis;

FIG. 20 is an expanded view of the data in FIG. 16, the $V_{min}$ voltage is indicated on the zero glucose concentration trace and glucose concentrations are assigned to each of the voltage traces after the zero glucose concentration;

FIG. 21 shows a linear regression plot of $V_{min}$ values, obtained from the data in FIG. 20, on the Y-axis versus reference glucose concentration on the X-axis;

FIG. 22a shows a graph of a counter electrode voltage on the left Y-axis and working electrode voltage on the right Y-axis vs. time in microseconds. The working electrode poise potential is +0.5 volts vs. a Ag/AgCl reference electrode and various voltage points and a function, $dV_W/dt$, are defined within the graph;

FIG. 22b shows a linear regression plot of the slope of the initial response of the working electrode, (dVw/dt), on the Y-axis versus reference glucose concentration on the X-axis;

FIG. 23 shows a linear regression graph of the time points, t $V_{min}$, on the Y-axis versus a corresponding RC time constant on the X-axis;

FIG. 24 shows a plot of the time (t $V_{min}$, see FIG. 1) on the Y-axis plotted versus reference glucose concentration on the X-axis;

FIG. 25a shows a linear regression graph of $1/tV_{min}$ (time data from FIG. 24) on the Y-axis versus reference glucose concentration on the X-axis;

FIG. 25b shows a linear regression correlation plot of measured glucose concentration (from slope and intercept data from FIG. 25a) on the Y-axis versus reference glucose concentration on the X-axis;

FIG. 26a shows a linear regression graph of the final counter electrode voltage, $V_f$, (see FIG. 1) on the Y-axis versus reference glucose concentration on the X-axis;

FIG. 26b shows a linear regression correlation plot of measured glucose concentration (from slope and intercept data from FIG. 27a) on the Y-axis versus reference glucose concentration on the X-axis;

FIG. 27 shows a graph of calculated RC time constants on the Y-axis, derived from equation 5, versus reference glucose concentration on the X-axis;

FIG. 28 shows a linear regression graph of 1/RC on the Y-axis (from FIG. 27) versus reference glucose concentration on the X-axis;

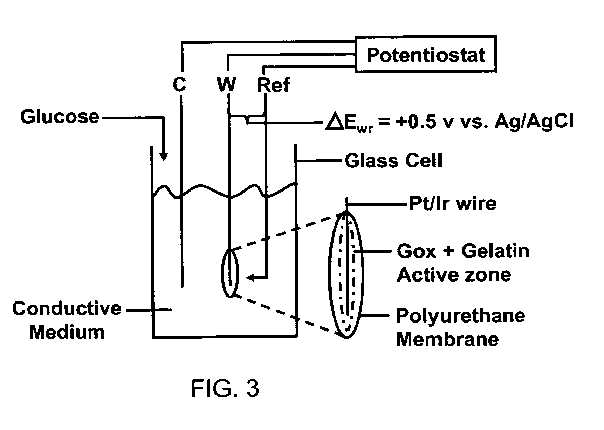

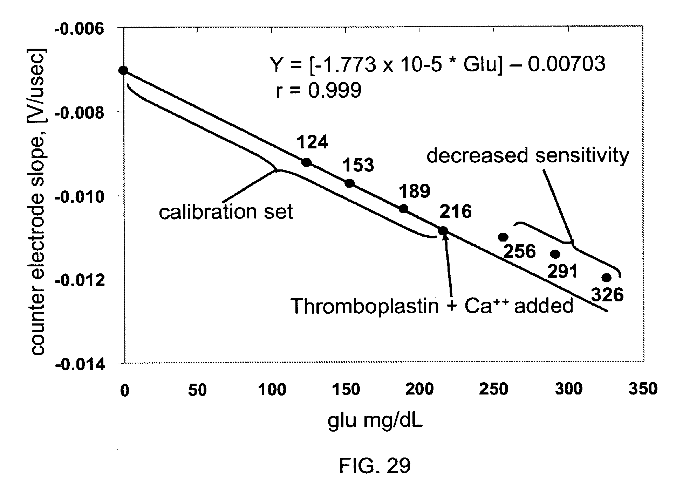

FIG. 29 shows a linear regression graph of the first four data points from the slope of counter voltage output versus time, $[dV/dt]_{max}$, on the Y- axis versus glucose concentration in citrated bovine plasma on the X- axis. The data was obtained with an in vitro electrochemical cell (see FIG. 3) having an amperometric glucose oxidase working electrode, platinum counter electrode and an Ag/ AgCl reference electrode in citrated bovine plasma. Data points (last 3) beyond 200 mg/dL glucose concentration, show a decrease in $[dV/dt]_{max}$ due to the addition of a clotting agent to the citrated bovine plasma simulating the effect of biofouling (fibrin formation) on the working electrode surface;

FIG. 30 shows a linear regression correlation graph between measured glucose on the Y-axis vs. reference glucose concentration on the X-axis. The slope and intercept data in FIG. 29 was used to calculate measured glucose concentrations. In FIG. 30, the error in calculated glucose concentration, caused by fibrin formation on the outer surface of a diffusion limiting barrier over the glucose oxidase sensor, is shown along with the error in calculated glucose concentrations before biofouling;

FIG. 31 shows a graph of the working electrode response ($i_W$, black trace) versus the first derivative with respect to time, gray trace $[di_{Wc}/dt]$ max, of the initial working electrode charging current ($i_{Wc}$) ;

FIG. 32 shows a working electrode response ($i_W$), from the application of a pulsed voltage between a counter electrode and a working electrode, where the peak current maximum response of a working electrode is denoted as

$[I_W]_p$. Various portions of the working electrode response are labeled as charging current, mixed current and Faradaic current;

FIG. 33a shows a linear regression plot of $[di_{Wc}/dt]^2{}_{max}* I_p$ (see FIG. 31) on the Y- axis versus reference glucose concentration on the X- axis is linear even in the presence of fibrin formation on the outside surface of the biosensor diffusion limiting barrier (see related data in FIG. 29) ;

FIG. 33b shows a linear regression correlation graph of measured glucose concentration on the Y-axis versus reference glucose concentration on the X-axis using the slope and intercept data in FIG 33a;

FIG. 34 shows a flow chart of how the present invention may be used to measure analyte concentrations in liquid samples from either counter electrode voltage or working electrode current response.

## DETAILED DESCRIPTION OF THE PREFERRED EMBODIMENTS

### DEFINITIONS

It is to be understood that the terminology used herein is for describing particular embodiments only, and is not intended to be limiting, since the scope of the present invention will be limited only by the appended claims.

As used herein and in the appended claims, the singular indefinite forms "a," "an," and the singular definite form, "the," include plural referents unless the context clearly dictates otherwise. Thus, for example, reference to a current transient includes a plurality of such current transients and reference to an analyte includes reference to one or more analytes and equivalents thereof known to those skilled in the art.

As used herein, the term "computing system" means a system comprising a micro-processor, an input device coupled to the micro-processor, an output device coupled to the micro-processor, and memory devices coupled to the micro-processor. The "input device" may be for example, a touchpad, a miniature keyboard, keypad, etc. "Output devices" may include a printer, a plotter, a computer screen, a wireless data transmitter, a data transmission cable (e.g., a USB cable) etc. "Memory devices" may include dynamic random access memory (DRAM), or read-only memory (ROM), FRAM etc. The memory device may include "computer code." The micro-processor executes computer code including algorithms for calculation of analyte concentrations. The memory device includes "input data." The input data includes input required by the computer code, such as sensor response data. The "output device" displays output from the computer code such as analyte concentrations. Memory devices may be used as a computer usable medium (or a computer readable medium or a program storage device) having a computer readable program code embodied therein and/or having other data stored therein, wherein the computer readable program code comprises the computer code. A "computer program product" (or, alternatively, an article of manufacture) of the computer system may comprise the computer usable medium or the program storage device. Any configuration of hardware and software, as would be known to a person of ordinary skill in the art, may be utilized to configure the computer system. The words "computing system," "recording system," and "analysis system" are understood to be synonymous herein.

As used herein, the term "slope" is defined as the change in the response of an electrode or electronic device per unit change in time or concentration of analyte. In the case of a glucose oxidase ("GOx") amperometric enzyme biosensor, the biosensor response is directly proportional to the glucose concentration and the slope is expressed as current/mg/dL or current/millimoles/Liter (mM). For example, the sensitivity or rate of change may be determined between two given time points or by linear regression of response vs. time or concentration.

"Equilibration period," "equilibration time," or "break-in period" refers to the time period required for an electrode response to reach a steady-state value.

As used herein, the terms "poise voltage" or "applied voltage" refer to a constant or floating electrical potential difference, respectively, between: (a) a working electrode and a reference electrode, represented as $E_{wr}$; or, (b) a counter electrode and a working electrode, represented as $E_{wc}$.

A "potentiostat" is used to supply a voltage between the working and counter electrodes. By means of a feedback circuit, the potentiostat varies the applied potential $E_{wc}$ to maintain a constant poise potential $E_{wr}$.

The term "diffusion limiting barrier" refers to a covering over a working electrode or counter electrode including a porous or semi permeable material, such as, for example, a polymeric material that limits diffusion of species into and out of the working electrode active zone. The diffusion limiting barrier also prevents migration of chemical species out of the biosensor, such as, for example, enzymes and mediators, or it may prevent the migration of unwanted components within tissue, cells or body fluid into the biosensor active zone, wherein, in either case, they may adversely affect the biosensor's response. The diffusion limiting barrier may also serve to limit the diffusion of a target analyte into the active zone, thus improving the linearity of the biosensor's response, or preventing saturation of the response. The terms "membrane," "semi permeable material," "semi permeable membrane," "coating," "barrier," "protective barrier," "diffusion limiting barrier," "diffusion limiting coating," or "barrier membrane" are generally understood to be synonymous herein.

Other definitions are described in the specification and defined within the context of their use.

This technology relates to devices and methods for measuring analytes dissolved in a conductive medium.

More specifically, the technology relates to, but is not limited to, electrochemical measurements that rely on the response of an electronic component within an electronic circuit, such as an operational amplifier, to changes in impedance between the counter electrode and working electrode of an electrochemical cell. This technology describes how functions of counter electrode voltage, such as the rate of change of the voltage output (slew rate), from an operational amplifier driving a counter electrode, is proportional to the concentration of an analyte at the working electrode.

The working electrode may be covered with a diffusion limiting barrier. An interface or space between the underside of a diffusion limiting barrier and a working electrode can create a small, confined volume where concentrations of ions, charge carrying by-products or conductivity enhancing species from chemical reactions and/or electrochemical oxidation or reduction of an analyte can change the conductance within the active zone of a working electrode. The diffusion limiting barrier can temporarily limit the flow of ionic or conductivity enhancing species away from the working electrode active zone across a diffusion limiting barrier into the bulk solution on the outside surface of a working electrode.

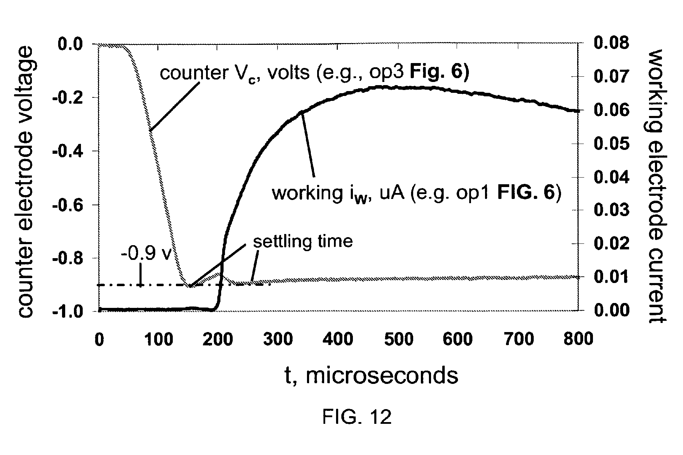

The temporary increase or decrease in conductance can be measured from the microsecond ($\approx 100 \times 10^{-6}$ seconds) response time of an operational amplifier (op amp) voltage output driving a counter electrode. The counter electrode voltage response occurs before the working electrode response. This technology demonstrates that the counter electrode op amp voltage output is proportional to analyte concentration. Before the working electrode can respond, the voltage output from the counter electrode op amp must reach a level that allows the working electrode voltage (e.g., versus a reference electrode) to reach a point where electrochemical oxidation or reduction can occur. The time to reach that voltage is dependent on an RC time constant that is principally due to the capacitance and resistance introduced by the electrochemical cell. For example, as shown in FIG. 12, the "settling time" of the counter electrode voltage is on the order of 200 microseconds, about the same time the working electrode response begins. Thus, the time period prior to the response from the working electrode is less that about 200 microseconds, preferably from about 20 to 150 microseconds, and most preferably from about 80 to 100 microseconds. Not only the rate of change of the voltage supplied to the counter electrode, but also the maximum voltage ($V_{max}$), minimum voltage ($V_{min}$), final voltage ($V_f$), as well as other functions of the counter electrode voltage vary in proportion to the concentration of analyte at the working electrode.

In general, the technology provides: an electrochemical cell including a plurality of electrodes; an analysis system with one or more operational amplifiers whose electrical properties change in proportion to the concentration of an analyte; and a computing system that determines and provides a read-out of concentration of an analyte or analytes.

This technology relates to measurements of dissolved analytes contained within a conductive medium or the interaction of a conductive medium, containing an analyte, with an electrical conductor. One embodiment of this invention includes an electrochemical cell. The minimum requirement for an electrochemical cell is that it has at least two electrodes defined as a working electrode and a counter electrode with a conductive medium between the two electrodes to allow completion of an electrical circuit. The working electrode is defined as the electrode at which electrochemical oxidation or reduction may  occur to produce a response in the form of a current, voltage or time that is proportional to analyte concentration. If the working electrode is the positive terminal (anode) of the cell, the counter electrode is the negative terminal (cathode) and vice versa.

The electrochemical cell may be interfaced to a system, including for example, a potentiostat for application of a voltage or current between the counter electrode and working electrode. The system can also include a computing or recording system that: (1) records input and output of the electrochemical cell, (2) stores data in memory, (3) performs calculations on the data and (4) visually displays the data or calculations on the data or analyte concentration.

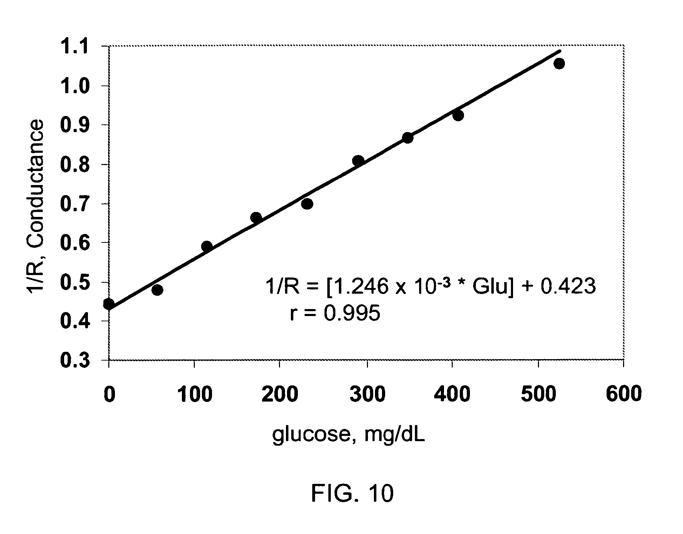

To obtain an unknown analyte concentration from a response measurement utilizes an equation that relates a response to analyte concentration. Most of the calculations described herein are linear and can be defined by the simple equation: y = mx +b; wherein y denotes the response, m is the slope of response versus analyte concentration, x is analyte concentration and b is the y-intercept of a plot of response versus analyte concentration. These plots are sometimes referred to as calibration plots. Examples of calibration plots are described in FIGs. 10, 17a, 17b, 18a, 18b, 19, 21, 22b, 23, 24, 25a, 25b, 26a, 26b, 27, 28, 29, 30, 33a, and 33b.

Using the slope and intercept from any of the graphs, an unknown analyte concentration is calculated as follows:

$$1.\ x = (y-b)/m.$$

In the graphs referred to above, the linear expression is shown as Y = mx +b. The slope and intercept from a graph of response on the Y-axis versus analyte concentration on the X-axis are substituted into equation 1 to calculate analyte concentration from a measured response. The slope and intercept are normally determined prior to analysis of an unknown analyte concentration by plotting response on the Y-axis versus known concentrations of analyte on the X-axis and using linear regression to determine the slope and intercept, or a line can be drawn between the response points versus analyte concentration which line stops at or crosses the Y-axis, that point on the Y axis yields the intercept, the response at zero analyte concentration, and the difference between at least two response points divided by the corresponding difference in analyte concentration yields the slope.

Another way to calculate analyte concentration is to use a single-point calibration. This requires that at least one concentration is known prior to the calculation of other unknown analyte concentrations. An example is the continuous measurement of glucose in vivo. To calibrate an in vivo glucose sensor requires an in vitro measurement of glucose, using for example a blood glucose meter, from a sample of blood, interstitial fluid (ISF) or other in vivo body fluid. The initial in vivo slope ($m_1$) from a single in vitro glucose measurement, $[G]_1$, from an in vivo sample, can be determined by:

$$2.\ m_1 = [i]_1/[G]_1.$$

Where $[i]_1$ refers to the current response to a known glucose concentration $[G]_1$ and the subscript 1 or n (n = 1,2,3...) indicates the response and analyte concentration are measured at the same point in time and calibration slopes beyond $m_1$ are noted as $m_n$ and a corresponding current response as $[i]_n$. Equation 2 assumes a zero intercept. In the case of an amperometric enzyme electrode, the response is a measured current and dividing the current by the glucose concentration yields a slope, $m_n$, equal to amps/mM (millimoles/Liter) or amps/mg/dL. Subsequent, unknown in vivo glucose concentrations may be calculated using the following equation:

$$3.\ [G]_n = \{[i]_n\text{-}[i]_1\}/m_1 + [G]_1,$$

where the subscript n denotes any glucose measurement taken after $[G]_1$ and $[i]_1$ serves as the intercept. The calibration process can be repeated at any time after measurement of $[G]_1$ and the new slope, intercept and single point measured glucose concentration used to calculate future unknown glucose concentrations.

The electrochemical cell may be a permanent or an integral part of the system or the electrochemical cell may be a separate unit that plugs into the system. In some embodiments, the electrochemical cell may have a third electrode known as a reference electrode. The system may include a plurality of electrochemical cells, electrodes or an array of electrodes.

Electrodes or combinations of electrodes may be immersed in a conductive medium in which analytes or other species are already present or to which analytes may be added in the form of solids, liquids or gases. The electrodes may be stored in the dry state and later activated by the addition of a conductive medium containing an analyte or the electrochemical cell may be exposed to the air whereby moisture in the air activates the electrodes for the measurement of analytes within air.

The working electrode of the electrochemical cell can have a biological component such as an enzyme, protein, antibody, antigen, RNA, DNA, DNA fragments, synthetic proteins, cells or cellular materials, and the like associated with, immobilized, entrapped or near its surface. In such case, the working electrode may be referred to as a biosensor. The biosensor may be used for in vitro or in vivo analyte measurements. An in vivo application can include electrodes or groups of electrodes such as electrochemical cells, wherein all or part are totally or partially in contact with eukaryotic or prokaryotic cells or tissue of humans, animals, or plants. Partially implanted sensor systems can include a plurality of in vivo electrodes with other electrodes ex vivo, as for example, on the skin surface. Together, the in vivo and/or ex vivo electrodes comprise one or more electrochemical cells.

This technology relates to electrochemical cells having at least two conductor(s) that, in combination, can complete an electrical circuit through which a voltage or current can flow. In addition, the cell(s) may contain a plurality of conductors that serve as working, counter, or reference electrodes. In one aspect of the invention, the electrochemical cell includes at least one conductor serving as a working electrode and another conductor, serving as a counter electrode. In another aspect of the invention, the conductors may be held within a fluid medium. The fluid medium may be in direct contact with each conductor or the conductors may be separated by a permeable material, with fluid surrounding both conductors and the permeable material. The permeable material allows the transport of ions and other low molecular weight species, dissolved in the external fluid medium, across the permeable material into the active zone. The fluid medium may be held in place, for example, with an enclosure such as plastic, glass, silicon, ceramic, polymers, adhesives or adhesive pads. The enclosure may also include a conductive gel that surrounds and contacts the conductors. The enclosure may also include body tissue either in vivo or ex vivo. An example of ex vivo tissue is the skin surface and an example of in vivo tissue is any subcutaneous or intradermal tissue.

Suitable conductors may be noble metals such as platinum, palladium, ruthenium, iridium, alloys such as platinum-ruthenium, platinum-iridium; other metals such as silver, titanium or alloys of metals such as titanium-aluminum, titanium-platinum, titanium-indium-cobalt, nickel alloys such as Inconel, Incoloy or Nitinol and other conductors such as graphite, carbon, glassy carbon, graphene, diamond, diamond- like carbon (DLC), single crystals, forms of carbon such as carbon nano- tubes, Fullerenes, nano- particles and the like. In addition, conductor materials may be semiconductors such as crystalline or amorphous silicon, doped silicon or other materials such as organic semiconductors. The conductor may also include an inert or non- conductive substrate such as plastic or a ceramic material upon which metal or other conductive materials are deposited by dipping, printing, plating, chemical vapor deposition or other means.

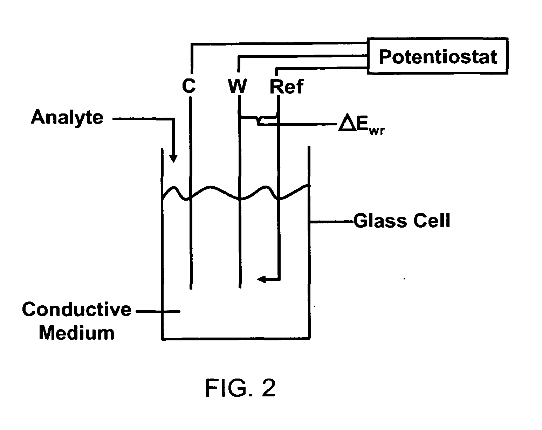

FIG. 2 shows a drawing of a three-electrode electrochemical cell with electrodes immersed in a conductive medium. The three electrodes include a working or sensing electrode, W, counter electrode, C, and reference electrode, R. For example, the conductive medium may contain dissolved ions derived from electrolytes such as potassium chloride, sodium chloride and/or buffer containing salts. An example of a suitable conductive medium is pH 7.4 phosphate buffered saline ("PBS") (available from Sigma-Aldrich). An example of a suitable reference electrode is a silver/silver chloride electrode.

FIG. 3 illustrates an example of an electrochemical cell wherein a working electrode is coated with a protein layer, such as glucose oxidase (GOx) and gelatin. In addition, the protein layer is covered by a second layer including a diffusion limiting barrier such as a polymer. For example, the polymer may be chosen from any polymer that can be applied by dipping, printing, spraying, spin coating, vapor deposition or in situ polymerization.

The diffusion limiting barrier allows the passage of small ionic and low molecular weight molecules such as sodium, potassium, chloride, phosphate, glucose, oxygen, etc, into and out of the active zone but excludes high molecular weight compounds such as proteins or cells. Examples of suitable polymers are silicones or polyurethanes that may be applied by dipping, printing, spraying or spin coating on the working electrode.

When a working electrode is covered, coated or enclosed within a diffusion limiting barrier, there is a small volume or interface between the inside surface of the diffusion barrier and the surface of the working electrode conductor. This space is referred to herein as the active zone (AZ), where chemical and electrochemical reactions can occur in close proximity to the electrode surface. The active zone may also serve as a vessel divided from the external bulk solution surrounding the working electrode, while still allowing diffusion into and out of the vessel.

This technology relies, in part, on the ability of a diffusion limiting barrier to temporarily slow diffusion of products, produced from chemical and electrochemical reactions within an active zone across a diffusion limiting barrier into a conductive medium or bulk solution surrounding the electrode. Products from chemical and electrochemical reactions, occurring within the working electrode active zone, are frequently charged or have high conductivity. The increase in conductivity reduces the electrical impedance between the working electrode and the counter electrode. These chemical and electrochemical reactions increase the conductance or admittance at the working electrode surface as reflected by the output voltage of an op amp driving a counter electrode.

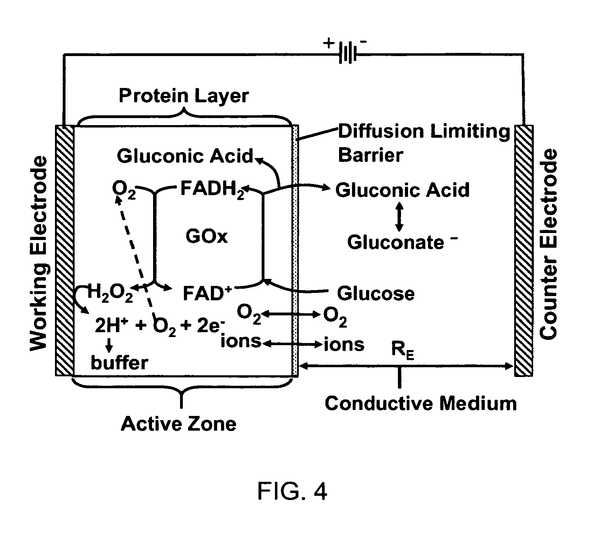

FIG. 4, shows an expanded view (not to scale) of the working electrode shown in FIG. 3. The protein layer, as defined in FIG. 4, can include a mixture of proteins both inert and active. An example of an active protein is an enzyme such as glucose oxidase (GOx). An example of an inert protein is gelatin. The protein mixture may be crosslinked, for example with glutaraldehyde or another crosslinking agent. The protein layer may be coated with a second layer of a diffusion limiting barrier in order to: (a) protect the protein coating on the electrode from the body's immune system; (b) limit diffusion of analytes and unwanted high molecular weight species within the external bulk solution into the active zone or, (c) limit the diffusion of products, proteins or high molecular species, within the active zone, out into the external bulk solution.

FIG. 4 further shows chemical and electrochemical reactions occurring within the active zone of a glucose oxidase working electrode in accordance with an embodiment of the present invention. Glucose from the external bulk solution diffuses across a diffusion limiting barrier into the active zone where it is oxidized by glucose oxidase to gluconic acid (or gluconolactone) and the two $FAD^+$ active sites in GOx are reduced to $FADH_2$. This is followed by oxidation of the $FAD_2$ groups by oxygen to produce hydrogen peroxide, 2 protons and oxidized GOx. These chemical reactions are catalytic and occur in the absence of an applied voltage. The electrochemical oxidation of hydrogen peroxide yields two protons ($2H^+$) and one oxygen molecule that can be recycled by the enzyme. The protons produced from the electrochemical oxidation of hydrogen peroxide produce a transient change in the pH, and thus conductance, within the active zone, before being consumed by buffer. The working electrode, active zone, and diffusion limiting barrier together constitute a half-cell of a two-electrode cell. The counter electrode half-cell in the right of FIG. 4 completes an electrical circuit with the working electrode on the left. Besides the working and counter electrodes, a reference electrode may be used to maintain a constant voltage between a reference electrode and working electrode.

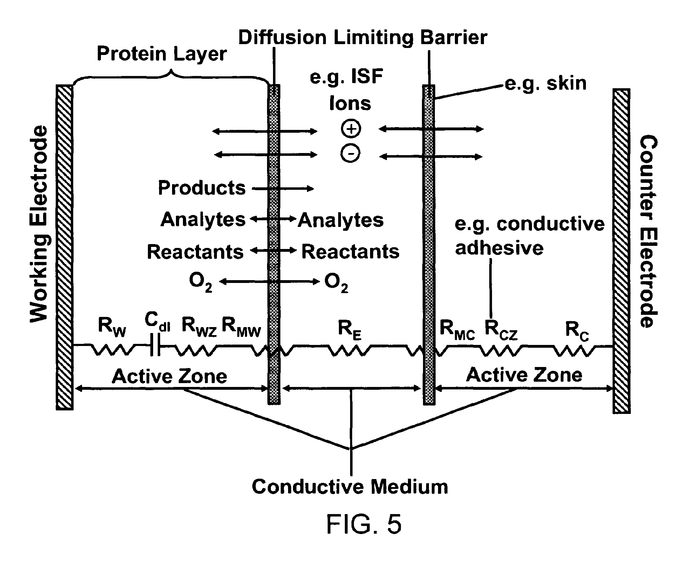

FIG. 5 provides another view, similar to FIG. 4, without the chemical and electrochemical reactions. FIG. 5 shows an example of the resistance and capacitance terms between the working electrode surface and the counter electrode surface. The double layer capacitance $C_{dl}$ exists at the surface of the working electrode. For example, the double layer capacitance may be in the range of about 1-50 microfarads ($10^{-6}$) per centimeter squared ($\mu F/cm^2$). Because there is a diffusion limiting barrier between the working and counter electrodes, the total resistance ($R_S$) between the working and counter electrodes is more complex. If both electrodes were bare conductors, $R_S$ would equal the conductive medium resistance, $R_E$, between the two conductors, including the intrinsic resistance ($R_w$ or $R_c$) of each conductor. In the presence of a diffusion limiting barrier, it is more constructive to compartmentalize and define the resistances between the working and counter electrodes that can contribute to the total resistance, $R_S$.

In FIG. 5, resistance components are labeled $R_W$, $R_{WZ}$, $R_{MW}$, $R_E$, $R_{MC}$, $R_{CZ}$ and $R_C$. The resistance term $R_W$ is the intrinsic resistance of the working electrode conductor, $R_{WZ}$ refers to the resistance of the matrix and fluid within the active zone of the working electrode, $R_{MW}$ is defined as the diffusion limiting barrier resistance. The term $R_E$ is defined as the resistance of the fluid between the outer surface of the counter electrode and the outer surface of the diffusion limiting barrier on the working electrode. Although not required for the practice of this invention, when a diffusion limiting barrier covers a counter electrode, the membrane resistance is defined as $R_{MC}$, the resistance within the active zone is denoted by $R_{CZ}$ and $R_C$ refers to the intrinsic resistance of the counter electrode conductor. With respect to the diffusion limiting barriers they need not be inert materials. For example, the diffusion limiting barrier could be a skin surface and the conductive medium as defined by $R_E$ could be a body fluid and the terms $R_{WZ}$ and $C_{WZ}$ could also be conductive gels. In the presence of a diffusion limiting barrier over the working and counter electrodes, the total resistance, between the working electrode and counter electrodes can be expressed as:

$$4. \quad R_S = R_W + R_{WZ} + R_{MW} + R_E + R_{MC} + R_{CZ} + R_C.$$

Referring to FIG. 5, the matrix within the active zone may include water, electrolytes, reactants, glucose, oxygen, proteins, glucose oxidase, enzymes, substrates, polymers, aqueous gels, mediators and products, such as hydrogen peroxide, hydrogen peroxide anions, gluconic acid, gluconolactone, anions, gluconate, cations, hydrogen ions ($H^+$), and other low molecular weight species. The terms $R_{MW}$ and $R_{MC}$ refer to the electrical resistance through a cross sectional area of a diffusion limiting barrier such as a polymer membrane. The diffusion limiting barrier resistance is a function of the type of material, its organic and/or inorganic content, thickness, density, hydrophilicity or hydrophobicity and porosity. The magnitude of $R_{MW}$ or $R_{MC}$ can range from tens of ohms to millions of ohms (Meg Ohms). The diffusion limiting barrier on the working electrode controls the diffusion of species such as glucose and oxygen into the active zone. If too much glucose diffuses into the active zone, there may not be sufficient oxygen, enzyme or a fast enough rate of mediator turnover to yield a linear response over a wide dynamic range. For an analyte such as glucose, the dynamic range may be 0-1000 mg/dL or 0-56 mmol/Liter (mM).

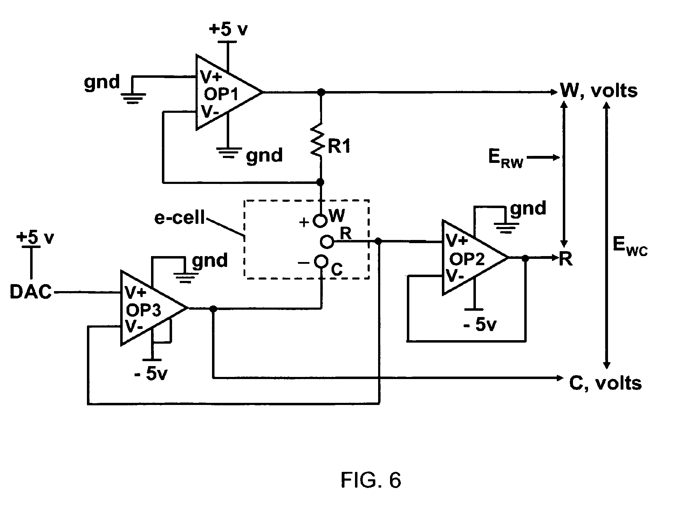

A potentiostat may be used to control the applied voltage or current to an electrochemical cell. FIG. 6 depicts a portion of an electronic circuit for an exemplary potentiostat that can be used to apply a voltage or current between the working and counter electrodes of an electrochemical cell. The working electrode can serve as the positive or negative terminal of the electrochemical cell. The combination of the working and counter electrode may also constitute a conductivity cell. In addition, the potentiostat can be programmed to apply various voltage waveforms between the counter and working electrodes.

In FIG. 6, the e-cell, within the area defined by the dotted line box, refers to three electrodes of an electrochemical cell that are operationally connected to the electronic circuit. The term OP1 refers to an operational amplifier used as a current to voltage converter for the working electrode output response. The calibrated voltage output of OP1 can be used to calculate an analyte concentration or the voltage output may be calibrated as a current response that mirrors the current input to OP1. Either way, voltage or current from the working electrode is proportional to analyte concentration. The second amplifier, OP2, is a voltage follower for the reference electrode and, in conjunction with OP3, the output voltage to the counter electrode is feedback adjusted to maintain a fixed voltage difference (poise potential) between the reference and working electrode.

In purely electronic systems, the rate of change of the output voltage of an op amp is dependent on the reactive components within the circuit. The maximum rate of change of the output voltage of an op amp is called the slew rate (SR), and is defined in units of volts per microsecond (V/$\mu$sec):

$$5. \quad SR = [dV/dt]_{max} = (i_{max}/C) = (E/RC),$$

where $[dV/dt]_{max}$ is the maximum rate of change or slope versus time of the output voltage of OP3 (FIG. 6) to the counter electrode, $i_{max}$ is the maximum current, C is the capacitance of the circuit and E is the voltage output to a counter electrode. For example, in the case of an external load such as an electrochemical cell, the voltage values of $V_{max}$, $V_{min}$ or $V_f$, derived from the counter electrode voltage profile (see FIG. 1), may be substituted for E and $R_S$ or the iR drop across the electrochemical cell can be substituted for R in equation 5. In electronic circuits, one of the primary factors controlling slew rate is the capacitance within the circuit. As capacitance (or impedance) increases, the slew rate decreases. If the op amp is internally compensated with a defined capacitance to enable unity gain stability, then the slew rate is dependent on the rate of charging and discharging of the compensation capacitor. If there is no defined internal capacitance, the slew rate will depend on internal parasitic capacitances. Internal capacitance may be on the order of tens of picofarads ($10^{-12}$ farads). The nominal or maximum slew rate is specified in the data sheet from the manufacturer of the operational amplifier. The slew voltage is usually linear with time, and for example, the rate of change can be measured between two time points defined as the time between about 10% of the op amp output voltage and 90% of the output voltage. In addition, the slew rate can also be determined by linear regression of the output voltage versus time (microseconds) or by rate of change in voltage between two time points:

$$6. \quad (V_2 - V_1)/(t_2 - t_1) = [dV/dt] = V' \text{ (see FIG. 1)}.$$

In the absence of an external load such as an electrochemical cell, if the nominal slew rate of an op amp is 100 V/$\mu$sec, the time to reach 1.0 volt would be about 0.01 micro-seconds ($\mu$sec) or $10^{-8}$ sec. Rather than attempting to measure a very fast slew rate using a very fast analog to digital converter (ADC) that introduces higher cost and higher power consumption, a more reasonable approach is to use a relatively slow slew rate op amp, for example 5 V/$\mu$sec or less. A slower slew rate will reduce the sampling rate necessary to obtain sufficient data for an accurate measurement of slew rate when an external load, such as an electrochemical cell, is present. For example, if the slew rate of the op amp was 1.0 V/$\mu$sec, a sampling rate of 1 MHz would provide one data point per $\mu$sec. If the slew rate were 0.1 V/$\mu$sec, a sampling rate of 1 MHz, would yield 10 data points per $\mu$sec. Even with a slew rate of 0.01 1V/$\mu$sec, the counter electrode response would still be sufficiently rapid to reach 1V in 100 $\mu$sec ($10^{-4}$ seconds).

The parameter that will determine the maximum voltage output to the counter electrode or the compliance voltage of a potentiostat connected to an electrochemical cell is the maximum supply voltage to the op amp(s). Many op amps are rated at $\pm$ 3 or 5 volts; however, in the instant invention, it is the resistance and capacitance (impedance) between the working and counter electrodes, that determines the rate of change of the output voltage to the counter electrode and the time ("settling time") to reach the voltage necessary to maintain a constant voltage between the working and reference electrode. For example, if the desired voltage at the working electrode is +0.5 volts versus a silver/silver chloride reference electrode (poise voltage), there would be available -2.5 volts of compliance (iR drop) from the op amp controlling the counter electrode voltage if the maximum supply voltage to the op amp was -3.0 volts. If the maximum supply voltage was -5.0 volts, the compliance voltage of the op amp would be -4.5 volts at a working electrode potential of +0.5 volts versus an Ag/AgCl reference electrode. Under most conditions, 5 volts of compliance may be sufficient; however, if the working electrode is implanted within body tissue, the resistance and capacitance between the working and counter electrodes may increase dramatically, depending on the cell geometry.

This increased impedance may surpass the ability of an op amp to drive enough output voltage to the counter electrode to overcome the increased iR drop between a counter electrode and in vivo working electrode. If the maximum applied voltage to overcome the impedance between the counter and working electrodes exceeds the compliance voltage of an op amp driving the counter electrode, both the counter electrode voltage and the working electrode response will reach a plateau and flatten out until the impedance or resistance of the cell between the counter electrode and working electrode decreases to a point that is within the compliance voltage of the op amp. For example, if the resistance between the working and counter electrodes is 1.0 M$\Omega$ ($10^6$ ohms), and the maximum current measured at the working electrode is 100 nA ($10^{-7}$ Amps), the iR drop would be -0.10 volt. If the desired working electrode poise voltage was +0.50 volts between the working and reference electrode, the voltage required at the counter electrode would be about -0.60 volts.

To understand the response of an amperometric enzyme working electrode, both chemical and electrochemical reactions must be considered. For example, in the absence of oxygen, glucose oxidase (its native form is oxidized) will oxidize glucose until all the enzyme is in its reduced form, at which point, the reaction stops. If oxygen or another mediator is present, the enzyme can be reactivated so the catalytic cycle can continue until the entire supply of the mediator is oxidized. If a continuous supply of oxygen is present, the enzyme catalytic cycle will continue until all the glucose is irreversibly oxidized or the supply of analyte or substrate is depleted. As shown below, these reactions occur in the absence of an applied voltage.

$$7. \quad \text{Glucose} + \text{GOx}:(\text{FAD}^+) \rightarrow \text{GOx}:(\text{FADH}_2) + \text{Gluconic acid (chemical)}$$

$$8. \quad \text{GOx}:(\text{FADH}_2) + \text{O}_2 (\text{M}_{ox}) \rightarrow \text{GOx}:(\text{FAD}^+) + \text{H}_2\text{O}_2 (\text{M}_{red}) - 2e^- \text{ (chemical)}$$

$$9.\quad H_2O_2 + H_2O \rightarrow HOO^- + H_3O^+ \quad pKa = 11.6 \quad base$$

(chemical)

$$10.\ \text{Gluconic Acid } (RCOOH) \leftrightarrow \text{Gluconate } (COO^-X^+)\ pKa = 3.7\ acid\ (chemical)$$

R = alkyl group

$$11.\ HOO^- + RCOOH \rightarrow RCOO^- + H_2O_2\ (acid\text{-}base\ reaction)$$

In equation 7 above, GOx represents glucose oxidase, the term ($FAD^+$ or just FAD) represents the native, oxidized form of Flavine Adenine Dinucleotide, the active site within GOx responsible for electron transfer and, ($FADH_2$) represents the reduced form of FAD. There are two FAD groups within the GOx enzyme. In equation 8, $M_{ox}$ represents the oxidized form of a mediator and $M_{red}$ represents the reduced form of a mediator and $2e^-$ represents the number of electrons transferred in the reduction of oxygen to hydrogen peroxide ($H_2O_2$). Equation 9 represents the dissociation of hydrogen peroxide in the presence of water to produce the basic anion $HOO^-$. In equation 10, the symbol $X^+$ represents a cationic species such a proton ($H^+$) or metal ion such as sodium ($Na^+$) or potassium ($K^+$). Gluconic acid is a weak acid in equilibrium with its anionic form as defined by the pKa of gluconic acid which is 3.7. In the absence of other effects, such as high pH, approximately 98% exists as gluconic acid and 2% exists as negatively charged gluconate. Equation 11 shows that the anionic form of $H_2O_2$ may also serve as a base that de-protonates gluconic acid to its anionic form gluconate. Equations 7-11 serve to show that within the active zone of the working electrode, in the absence of an applied voltage, the conductivity within the active zone can vary due to chemical reactions. This phenomenon can explain why the slew rate of the counter voltage, which occurs before electrochemical oxidation at the working electrode, is proportional to glucose concentration.

Referring to equation 8, by continually regenerating the oxidized mediator ($M_{ox}$) from ($M_{red}$), the catalytic cycle can continue. For example, the reduced form of oxygen ($M_{ox}$) is hydrogen peroxide ($M_{red}$). If there is a way to regenerate oxygen from hydrogen peroxide, the supply of dissolved oxygen can be replenished such that the concentration of oxygen in the bulk solution need not be present in high excess. The yield of oxygen from the electrochemical oxidation of hydrogen peroxide need not be 100%; however, enough may be regenerated to augment the supply of oxygen from the external bulk solution surrounding the working electrode, thereby reducing oxygen limitation at high glucose concentrations.

Hydrogen peroxide can be oxidized to oxygen through the use of a platinum electrode in an electrochemical cell. In the presence of an aqueous electrolyte solution and a platinum working electrode poised at a potential that causes catalytic oxidation of hydrogen peroxide (e.g. +0.4 to +0.6 volts vs. Ag/AgCl), oxidation of hydrogen peroxide causes a current to flow, the magnitude of which is directly proportional to the concentration of hydrogen peroxide generated and the concentration of an analyte such as glucose.

If a platinum electrode is associated with or has bound to its surface an enzyme such as glucose oxidase; in the presence of glucose and dissolved oxygen, hydrogen peroxide will be generated in proportion to the mass of glucose oxidized. Current generated from the electrochemical oxidation of hydrogen peroxide is directly proportional to the mass concentration (molality) of glucose consumed by the enzyme reaction. In general, this is a characteristic of amperometric enzyme working electrodes, where the concentration or activity of the analyte (e.g. glucose) is indirectly measured by the oxidation or reduction of a byproduct of the reaction of analyte with the enzyme (e.g. $H_2O_2$). This byproduct can be a reduced or oxidized mediator and, in the case of oxidase enzymes, the reduced mediator is either hydrogen peroxide and/or other mediator ($M_{red}$). In the presence of an applied voltage the oxidized form of the mediator is regenerated so the oxidation-reduction cycle continues as shown in equations 12 and 13 below:

$$12.\ H_2O_2\,(M_{red}) \rightarrow 2H^+ + O_2\,(M_{ox}) + 2e^-\ (ne^-)\ (electrochemical\ oxidation)$$

$$13.\ [M^{+2}]_{red} \rightarrow [M^{+3}]_{ox} + 1e^- (ne^-)\ (\text{electrochemical oxidation})$$

Equation 13 is a simplified expression for the turnover of mediator.

Mediators ($M_{ox}$ or $M_{red}$) are small molecules that can either oxidize or reduce the corresponding reduced or oxidized active site(s) within an enzyme by shuttling electrons between the enzyme and an electrode surface. Enzymes are typically large proteins having a three-dimensional structure. The active site of the enzyme may be buried within the enzyme's three-dimensional structure and not subject to direct electrochemical oxidation or reduction because the distance of the enzyme active site from the electrode surface is greater than the distance associated with direct electron transfer (less than about 20 Angstroms $\approx 2 \times 10^{-9}$ cm). The oxidized or reduced form of the mediator is small enough to diffuse into the active site of the enzyme, accept or give up electrons, and return to the working electrode surface to be reduced or oxidized electrochemically; thereby, recycling the mediator to its active form. The electrode response generated, in the form of a current or voltage, from the electrochemical oxidation or reduction of the mediator, is directly proportional to the mediator concentration and the concentration of an enzyme specific substrate such as glucose. Mediators can include the oxidized or reduced form of metal ions such as $Fe^{+3}/Fe^{+2}$ found in compounds such as ferriand ferro-cyanides, organo-metallic compounds such as ferrocenes, a quinone/hydroquinone couple or neutral molecules such as oxygen ($O_2$), the native mediator for glucose oxidase.

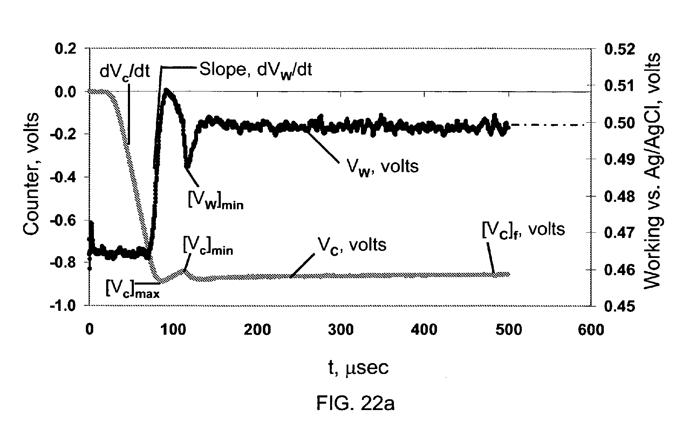

The electrochemical oxidation of hydrogen peroxide, shown in equation 12, produces 2 protons ($2\ H^+$) that can result in a transient change in pH, within the active zone, and thus a transient increase or decrease in the conductance within the active zone. Referring to FIG. 22a, the counter electrode voltage $[V_c]_{min}$ indicates where the contribution from increased charge generation is at a maximum. To the left of $[V_c]_{min}$ charge is increasing, while to the right of $[V_c]_{min}$ charge is decreasing.

Changes in the local pH or other ions may be temporary due to diffusion away from the electrode surface or the neutralization of hydrogen ions by buffer within the active zone, such as phosphate buffer. Whether the increase in conductance within the active zone is due to gluconate, gluconic acid, hydrogen peroxide anions or hydrogen ions, the slew rate of the op amp controlling the voltage to the counter electrode will increase when glucose is higher and decrease when glucose is lower.

BIOFOULING

As opposed to measurements of glucose concentration in vitro, if a working electrode is implanted in vivo, the body's immune system may recognize the electrode as a foreign body. The resulting inflammatory response, also known as biofouling, may be acute (mild, resolves quickly) or chronic (moderate to severe, longer term). Proteins and cells can attach to the outer surface of the diffusion limiting barrier and recruit other cells to dissolve, eject or remove the foreign body. The magnitude of the foreign body response is a function of the size of the implanted sensor and the biocompatibility of the outside surface of the implanted sensor. Therefore, more or less protein, cells or fibrinous material can accumulate on the outer surface of an implanted working electrode thereby increasing electrical resistance and reducing the surface area for diffusion of reactants such as oxygen and glucose into the active zone. The term $R_{Bio}$ is used to denote the electrical resistance of the material (e.g. cells, protein, fibrin, etc.) adhering to the outer surface of the implanted sensor.

The foreign body response can result in loss of working electrode sensitivity over time, yielding inaccurate measurements of analyte concentrations. For example, in vivo glucose electrodes require frequent, ex vivo re-calibration using an in vitro method such a blood glucose meter (BGM). This re-calibration process may require the user to frequently prick their finger and measure blood glucose concentration using a test strip and blood glucose meter and enter the resulting glucose concentration into the readout device or monitor to reset calibration parameters.

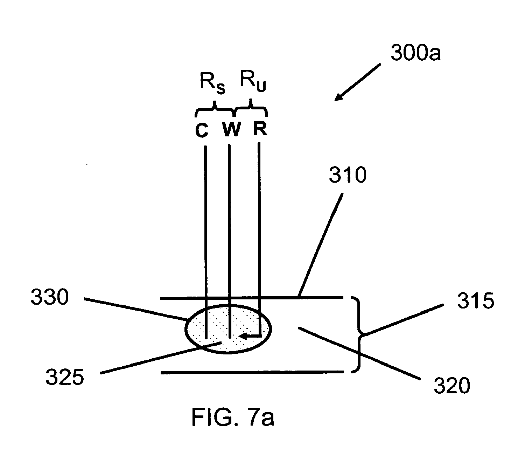

FIG. 7a shows an example of an in vivo configuration for an implanted three-electrode cell (300a) including an amperometric enzyme working electrode (W), a counter electrode (C), a reference electrode (R), a skin surface (310), a skin thickness (315), subcutaneous tissue and interstitial fluid (ISF) (320), an active zone (325), a diffusion limiting barrier (330), resistance ($R_s$) between the working and counter electrodes and uncompensated resistance ($R_u$) between the working and reference electrodes.

In FIG. 7a, all three electrodes are implanted within subcutaneous tissue (320) and are encapsulated within a diffusion limiting barrier (330). The application of a diffusion limiting barrier over the electrodes leaves a small space or interface (325) called the active zone, between the inside surface of the diffusion limiting barrier and the working or counter electrode, that serves as a path of fluid communication between the implanted electrodes and surrounding body fluid. This cell geometry is near the ideal configuration for reducing $R_S$. In the ideal electrochemical cell, the counter and working electrodes are as close together as possible to minimize $R_S$, and the reference electrode is as close as possible to the working electrode, without shielding the working electrode surface, to minimize $R_u$, the uncompensated resistance between the working electrode and reference electrode. Even with ideal cell geometry, the act of implantation of a three-electrode electrochemical cell may still elicit an inflammatory or biofouling response to the implanted sensor(s).

The implanted electrodes in FIG. 7a represent an enclosed electrochemical cell and the resistance between the working and counter electrodes will be dependent not only on the ionic strength of the fluid medium within the active zone, but also the mass of glucose within the active zone. The volume of the active zone may be fractions of a milliliter, for example nanoliters, and even though glucose is a neutral molecule and does not directly contribute to the electrolyte concentration it can reduce conductance by excluding ions, such as electrolytes, from the active zone. This may explain why the $V_{max}$ voltage is inversely proportional to glucose concentration. As glucose increases, the conductivity within the active zone may decrease, even though chemical reactions produce charge carriers. One would expect the $V_{max}$ voltage to become less negative when the glucose concentration is increasing Indirectly, the glucose dependency of the ionic strength or conductivity is due to the generation of transient charge carriers from the enzymatic oxidation of glucose (equations 7-11) and electrochemical oxidation of hydrogen peroxide within the active zone (equation 12). As a result, the number of charge carriers increases when glucose is high and decreases when glucose is low.

The above phenomenon may not exist at a working electrode without the presence of a diffusion limiting barrier; especially, if the fluid surrounding the working electrode is moving or stirred. Under mixing conditions any conductive enhancing species, generated at the working electrode surface, may be rapidly washed away by the external fluid surrounding the working electrode and the transient increase in conductivity may be too short or non-existent as to not be observed in the output voltage of the an op amp driving the counter electrode. In the case of static fluid surrounding the non-encapsulated or bare conductance electrodes (W+C), equilibrium may be very slow (minutes) and complicated by convective mixing, temperature or other movement artifacts. This type of cell would not be ideal for observing the changes in conductance at the working electrode surface as described in this invention or suitable for implanting within tissue.

In the presence of a diffusion limiting barrier on the working electrode and the resulting increase in the concentration of charge carriers within the active zone, the slew rate of the op amp driving the counter electrode voltage may be proportional to analyte concentration. The amount of biofouling on the outside surface of the diffusion limiting barrier will impact the mass of glucose entering the active zone which will decrease the number of charge carriers generated. The increase in conductivity in the presence of increasing glucose activity is counteracted by reduced mass transfer of glucose caused by biofouling.

For example, if an in vivo sensor was calibrated within the first hour following implantation, the biofouling layer may not be as extensive compared with biofouling hours later. In the former case, the calibrated sensitivity of the working electrode may be higher than the latter case, when there may be more biofouling. If biofouling has increased and the same calibration parameters determined from the first calibration are used to calculate glucose concentration at a later time, the calculated glucose concentration will be inaccurate. In order to reduce this inaccuracy, re-calibration is necessary. The extent of the inflammatory response and subsequent biofouling usually results in a lower measured glucose concentration versus a glucose measurement in the absence of biofouling. However, a chronic inflammatory response could result in edema around the implant site which increases the fluid volume (ISF or lymph) surrounding the implanted sensor(s). The increased fluid volume may contain a higher concentration of glucose than that found in normal tissue ISF. As a result, the calculated glucose concentration at the implant site may be higher than that found in non-inflamed tissue.

If the inflammatory response is chronic, the re-calibration process may need to be performed on a regular basis throughout the implant period because biofouling caused by increasing inflammation may not reach a steady state and may increase to the point where the sensor becomes enclosed within a thick fibrin capsule such that sensor response is lost. If the inflammatory response is acute and resolves or reaches a steady state within hours, then a number of re-calibrations may be necessary over the first few hours or days, but beyond that time, further re-calibrations may not be necessary because biofouling has reached a steady-state.

As shown in FIG. 7a, when all three electrodes are implanted within tissue, a way to determine the effect of biofouling on the outside surface of the diffusion limiting barrier is to determine how biofouling is affecting the working electrode response compared to a standard or comparison sensor that has no biofouling. In practice, this is difficult to achieve because the comparison sensor would need to be implanted in close proximity to the in vivo working electrode and therefore susceptible to the same biofouling effects (termed $R_{Bio}$) as the working electrode.

In the case of $R_{Bio}$, the build up of proteins, cells or fibrin on the outside surface of the diffusion limiting barrier may increase resistance, but the reduction in surface area for analyte diffusion will also have an effect on $R_{MZ}$. Regardless of whether there is biofouling present, the slew rate response of OP3 driving the counter electrode voltage in FIG. 6 is nevertheless dependent on the conductance or admittance within the active zone. The presence of biofouling on the outer surface of a diffusion limiting barrier increases the total resistance ($R_s$) while at the same time reducing glucose mass transport. The result of these effects is a reduction in the conductance or admittance within the active zone, thereby reducing the slew rate voltage from a counter electrode op amp. The slew rate can be viewed as a lumped term expression for describing the combined effects of changing cell impedance, analyte concentration, biofouling and the "background" impedance of the electrochemical cell.

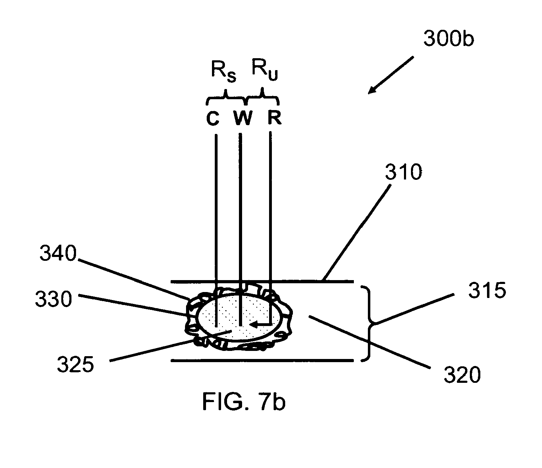

FIG. 7b depicts how biofouling can affect the resistance across the diffusion limiting barrier in FIG. 7a. In FIG. 7b, electrochemical cell (300b) includes a working electrode (W), a counter electrode (C), a reference electrode (R), a skin surface (310), a skin thickness (315), tissue and interstitial fluid (320), an active zone (325), a diffusion limiting barrier (330) and a biofouling layer (340), resistance ($R_S$) between the working and counter electrodes and an uncompensated resistance ($R_U$) between the working and reference electrodes. Biofouling layer (340) includes bound proteins, cells, fibrin or other physiological material on the outside surface of the implanted electrodes. This is in contrast to FIG. 7a, which does not include biofouling. The effect of biofouling on the outer surface of the implanted electrodes in FIG. 7b will limit the surface area for glucose transport, thus lowering the mass of glucose entering the active zone. This will reduce the response of the working electrode. In order to account for this loss in sensitivity, the in vivo working electrode must be re-calibrated using an in vitro method such as a blood glucose meter or other in vitro reference method.

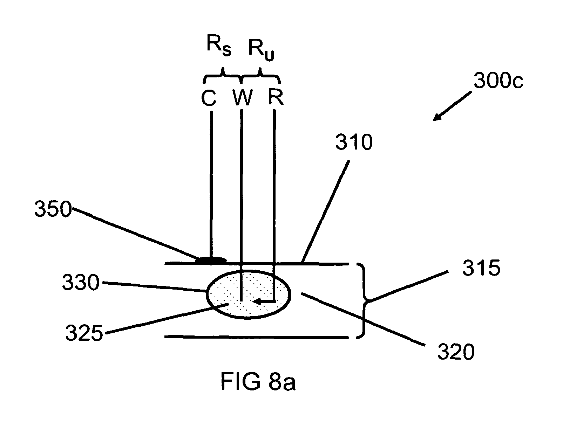

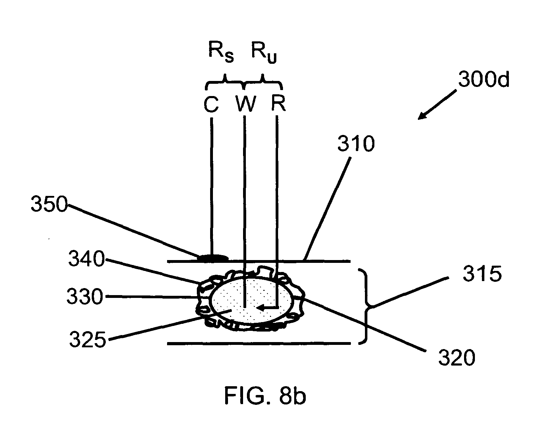

FIG. 8a illustrates a slightly different cell configuration compared to FIGs. 7a and 7b. In FIG. 8a, electrochemical cell (300c) has the working electrode (W) and reference electrode (R) implanted beneath a skin surface (310), a skin thickness (315), tissue and ISF (320), active zone (315), diffusion limiting barrier (330) and a counter electrode (350) that makes electrical contact with the skin surface. The working (W) and reference electrodes (R) are encapsulated within the same diffusion limiting barrier (330) thus minimizing $R_U$; however, the total resistance $R_S$, between the working and counter electrodes is more complex. FIG. 8b shows electrochemical cell (300d) having a working electrode (W) and reference electrode (R) implanted beneath a skin surface (310), a skin thickness (315), tissue and ISF (320), active zone (315), diffusion limiting barrier (330) and a counter electrode (350) that makes electrical contact with the skin surface the addition of biofouling (340) on the outer surface of the implanted electrode(s). In addition to the resistance terms previously identified above, there are additional terms to account for biofouling, $R_{Bio}$, on the outside surface of a diffusion barrier and the resistance through the skin and subcutaneous tissue designated as $R_{Skin}$. In this case, the total resistance between the working and counter electrodes can be expressed as:

$$14.\ R_S = R_W + R_{WZ} + R_{MW} + R_{Bio} + R_E + R_{Skin} + R_{MC} + R_{CZ} + R_C$$

Equation 14 is an expression for the total resistance, ($R_S$), between the counter electrode surface (C) on the skin surface and the implanted working electrode surface (W). The terms $R_W$, $R_{WZ}$, $R_{MW}$, $R_E$, $R_{MC}$, $R_{CZ}$ and $R_C$ are as previously described in equation 4. The major contributors to $R_S$ in equation 14 are $R_{MW}$, $R_{Bio}$ and $R_{MC}$. However, the terms $R_W$ and $R_C$ are constants and $R_{MW}$, $R_E$ $R_{MC}$ and $R_{SKIN}$ may be relatively constant once the implanted sensor(s) equilibrates with surrounding tissue and fluid (ISF). Equation 14 can be simplified by combining the "constant" resistance into $R_{Sys}$ and rewriting equation 14 as:

$$15.\ R_S = R_{WZ} + R_{Bio} + R_{CZ} + R_{Sys}\ ;$$

and the "constant" terms in $R_S$ are defined as:

$$16.\ R_{Sys} = R_W + R_{MW} + R_E + R_{Skin} + R_{MC} + R_C.$$

The major resistance terms in $R_{Sys}$ are likely the resistance across the diffusion limiting barriers, $R_{MW}$ and $R_{MC}$. The impedance ($R_{Skin}$) measured between a skin surface electrode and a counter or working electrode is, to a large degree, dependent on the contact resistance between the skin electrode and the skin surface. For example, when electrocardiogram (ECG) electrodes are placed on the skin, a conductive adhesive over the ECG electrode ensures good electrical contact between the electrode and skin surface. These types of conductive adhesives are polymeric materials that have some small resistance; however, the resistance measured between the electrode surface and the skin surface is usually in the range of 0.01-0.2 Meg Ohms ($M\Omega$, $10^6$). Referring to FIG. 5, the diffusion limiting barrier, $R_{MC}$, near the counter electrode may be a thickness of skin and the term $R_{CZ}$ may be a conductive adhesive between the counter electrode and a skin surface.

For example, if a polyurethane membrane is used as a diffusion limiting barrier, the polymer itself can have a significant cross sectional resistance that could be on the order of Meg Ohms ($M\Omega s$), somewhat dependent on the ratio of hydrophilic to hydrophobic domains and porosity. Thicker diffusion limiting barriers or those with tighter pore structure yield higher resistances. There is a compromise to be made between good diffusion control and increased diffusion limiting barrier resistance. Better diffusion control is obtained at the expense of increased diffusion limiting barrier resistance. The total resistance term $R_S$, in equation 14 could be in the range of 1-5 M$\Omega$. For example, if $R_S$ was 5 M$\Omega$ and the maximum current measured is 100 nA (1 nA = $10^{-9}$ Amps) the iR drop between the working and counter electrodes would be -0.50 volts. If the desired poise potential was +0.50 volts (W vs. Ag/AgCl reference electrode), the required final voltage at the counter electrode would need to be about -1.0 volts.

PULSED VOLTAGE MEASUREMENTS

In order to continuously measure the slew rate of the output voltage of an op amp driving a counter electrode and obtain multiple data points over relatively short time periods, the voltage applied between a counter electrode and a working electrode is preferably pulsed or applied intermittently using a waveform generator. For example, various kinds of waveforms (sine, triangular, square, etc.) may be applied between the working and counter electrodes. The waveform may be applied for a certain time period and then turned off. Application of a waveform provides the opportunity to make multiple measurements of slew rate over short time periods. If there was no periodic waveform applied, the "final" or equilibrium voltage (e.g., $V_f$) at the counter electrode would still be proportional to analyte concentration; however, the slew rate measurement could only be made once, upon the initial application of the applied voltage between the counter and reference electrodes. Because the applied voltage is not pulsed, multiple rapid (< about 200 $\mu$sec) measurements of analyte concentration from counter electrode voltage measurements may not be possible.

FIG. 9a shows a series (400a) of intermittent square wave voltage pulses having a total period ($\tau_t$), with a voltage on-time ($\tau_1$) and a voltage off-time ($\tau_2$) where:

$$17.\ \tau_t = \tau_1 + \tau_2.$$

Referring to FIG 9a and equation 17, if the total period $\tau_t$ is 5 seconds, the voltage on-time ($\tau_1$) may be 0.3 seconds with an off-time ($\tau_2$) of 4.7 seconds, or both the on-time and off-time may be 2.5 seconds, or any combination of ($\tau_1$) and ($\tau_2$) that adds up to the total period ($\tau_t$). In FIG. 9a, the application of a square wave voltage pulse, (410), gives rise to a working electrode voltage maximum (420), which remains constant throughout $\tau_1$. The voltage pulse also creates a working electrode response as a current vs. time transient, (430), with a maximum at (420). Under ideal conditions, within $\tau_1$, the concentration of analyte at the electrode surface drops to near zero before the next pulse. Between pulses, the off-time period $\tau_2$ allows the concentration of glucose oxidation products (equations 7-11) to increase so when another waveform pulse is applied there is another voltage vs. time response from the counter electrode and a new slew rate for the counter electrode response may be calculated. In addition, beyond the slew rate measurement, the working electrode peak response ($I_p$) or any current value beyond $I_p$, along the falling current transient ($i_t$) (430), is directly proportional to glucose concentration. When the voltage is turned off, at the end of $\tau_1$, the poise voltage, (440), falls to zero or the open circuit potential.

Within the time period $\tau_2$, there may be significant capacitance and resistance between the working and counter electrode, even though there may not be an applied voltage, it may take some time for the voltage to "bleed off" the working electrode due to an RC time constant. This possibility is illustrated in FIG. 9b wherein, the voltage drop (440) in FIG. 9a is now replaced by prolonged voltage decay (450).

The voltage output of the op amp driving the counter electrode is dependent not only on the reactance of components within the electronic circuit(s) it is also dependent on the reactive components introduced by the electrochemical cell (external load). Taken in total, there may be present both active (capacitance, inductance) and passive components (resistance) between the counter electrode and working electrode of an electrochemical cell. Due to reactions of an analyte within the active zone, the total resistance term ($R_s$) will have a variable contribution to the total conductance or admittance.

Conductance is a measure of the ease with which electrons flow through a conductor and is expressed as the reciprocal of resistance or I/R ($\Omega^{-1}$, or Siemens). When there are present, within the circuit, both resistive and reactive components, such as capacitors and inductors, the interconnection of these devices can no longer be analyzed by scalar quantities such as resistance or conductance, rather the complex impedance, Z, is a better description of the combined effects. Impedance is expressed as:

$$18.\ Z = R + iX,$$

where R is the real or scalar component and iX is the imaginary, complex term that accounts for the reactive components. The analogous term to conductance, 1/R, is 1/Z defined as admittance.

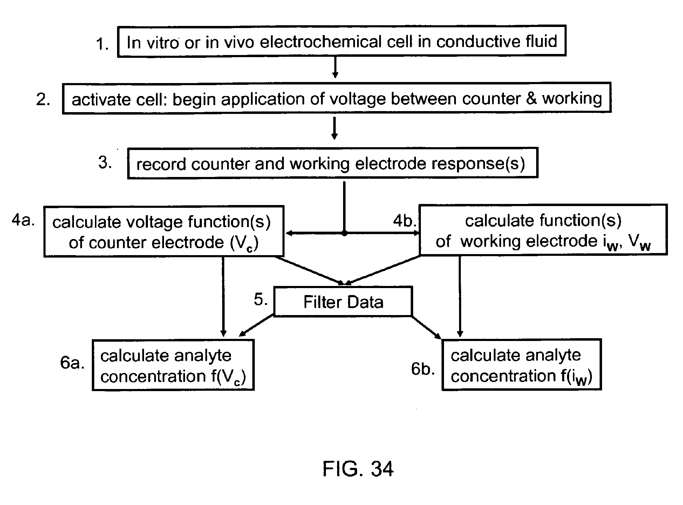

FIG. 34 shows a flow chart of how an embodiment of the present invention measures analyte concentrations in liquid samples:

Referring to FIG. 34, in

Step 1: Electrodes or electrochemical cell(s) are placed in a conductive medium (for example, dissolved electrolytes, buffer, water, body fluid, etc.). Analytes may be already present within the conductive medium or analytes may be added to the conductive medium. A combination of electrodes or electrochemical cells may be in the form of (a) dry or wet strip onto which analyte samples are added, (b) combinations of electrodes suspended in a conductive medium or (c) electrochemical cell(s) that are placed completely or partially in vivo and surrounded by body fluid;

Step 2: Energy is applied between a counter electrode and working electrode of an electrochemical cell (Step 1.) in the form of a voltage or current. The energy may be pulsed at regular time intervals.

Step 3: The response of the counter electrode and/or working electrode of an electrochemical cell is recorded. One response may be the op amp voltage input to the counter electrode. A second response may be the output response from a working electrode.

Step 4a: A function of a counter electrode voltage is measured or calculated. With respect to a counter electrode, the function may be in the form of a single voltage point, $V_{max}$, $V_{min}$, $V_f$, an averaged voltage, a slope, rate of change, intercept, maximum, minimum, steady state or other function of the counter electrode voltage such as t $V_{max}$, t $V_{min}$, RC or 1/RC. The function of the counter electrode voltage may be filtered in Step 5 or used directly to calculate an analyte concentration as in Step 6a.

Step 4b: A function of a working electrode response may be measured or calculated, such as Ip, $dV_w/dt$, $di_w/dt$ or $i_w$. With respect to a  working electrode response, the function may be in the form of a single or averaged voltage, a single or averaged current, a slope, rate of change, intercept or other function of a working electrode response. The function of the working electrode response may be filtered as in Step 5 or used directly to calculate an analyte concentration as in Step 6b.

Step 5: Various filters such as low and high pass filters may be used to smooth the data and remove outliers or noise spikes. Additionally, the data may be adjusted for drift caused by biofouling or other processes.

Step 6a & 6b: Single or multiple responses or functions of either a counter or working electrode may be used to calculate analyte concentrations. The single or multiple responses or functions of the responses may be averaged. The functions or responses may be filtered before or after averaging. Single point or averaged measurements may be used to calculate analyte concentrations from equations relating response to analyte concentration or the averaged functions or responses may be corrected for signal drift and then used to calculate an analyte concentration. In addition, analyte concentrations calculated from the counter electrode response(s) may be averaged with corresponding analyte concentrations calculated from working electrode response(s) to yield a redundant, more accurate indication of analyte concentrations.

The use of the technology described herein does not require complex impedance calculations or techniques such as impedance spectroscopy to determine how cell impedance affects the measurement of analyte concentrations. The measurement of slew rate (dV/dt) from the input voltage to the counter electrode encompasses the same information and is simpler to determine. The voltage slewing at the counter electrode finishes before there is a visible change in response from the working electrode (about 200 μsec, in FIG. 12). As a result, slew rate measurements from a counter electrode are not as noisy versus measurements of a working electrode current or voltage response (comparison shown in FIG. 22a). The response of an electronic component in an electrical circuit, such as an operational amplifier, is inherently less noisy than the current response of an enzyme based working electrode complicated by diffusion and biological processes; especially, in an in vivo environment.

In contrast to intermittent voltage application, if the voltage applied between the counter and working electrodes is continuous or steady-state, one may only observe the steady state voltage of the counter electrode (for example, $V_f$) to vary in proportion to the glucose concentration due to increases and decreases in conductance between the working and counter electrodes. In addition, when a steady state voltage is applied, the added information from the slew rate of an operation amplifier, controlling the voltage to the counter electrode, is not available because the op amp slewed only once the first time the potential between the working and counter electrodes was applied.

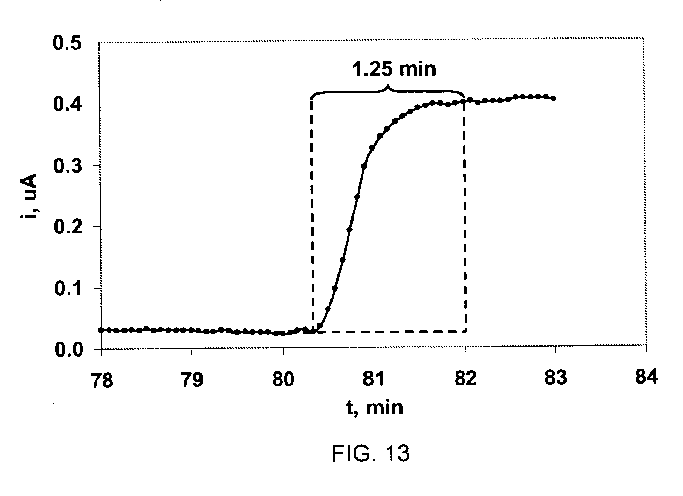

Prior art amperometric electrodes such as amperometric enzyme electrodes used to measure substrates such as glucose and other analytes, it is the response of the working electrode to changes in substrate or analyte concentration that is measured, and is most often current. In vitro working electrode measurements, taken after the addition of a sample of analyte, it is the steady state response of the working electrode that is measured and used to calculate analyte concentration. In the present case, the steady state response time of the working electrode is defined as the time to reach an equilibrium state. This response time is measured in minutes as opposed to microseconds for counter electrode voltage measurements. An example of the in vitro measurement of a working electrode response time of an amperometric enzyme electrode in pH 7.4 PBS is shown in FIG. 13 wherein the time to reach a steady state response is estimated to be 1.25 min.

As opposed to in vitro response times, in vivo response times are expected to be comparatively longer and more difficult to measure. The in vivo measurement of working electrode response time is difficult to determine due to the lack of equilibrium. Dynamic changes occurring in tissue fluid adjacent to the implanted working electrode, physiological lag time associated with glucose transport across the endothelium of capillaries into interstitial fluid, cellular consumption of the analyte and biological electrical noise can result in a non-steady state response. In vivo, the concentration of substrates or analytes, such as glucose, may be at a steady state for only brief periods of time; however, not knowing contributions from other processes, it is very difficult to parse the lag-time of the electrode from other in vivo dynamic processes.

The measurement of current or voltage from a working electrode response is more complex than measuring an independent property of an electronic component such as the slew rate of an operational amplifier. In addition, the working electrode response of amperometric enzyme electrodes can be dependent on a number of factors such as noise caused by high impedance, roughness of the working electrode surface, non-uniform current distribution, non-uniform coatings, flow rate of substrate around the working electrode and movement artifacts or muscle contractions. An increase in the amount of diffusion control results in a reduced diffusion coefficient, an increase in resistance across the diffusion barrier, decrease in substrate diffusion and an increase in the response time.

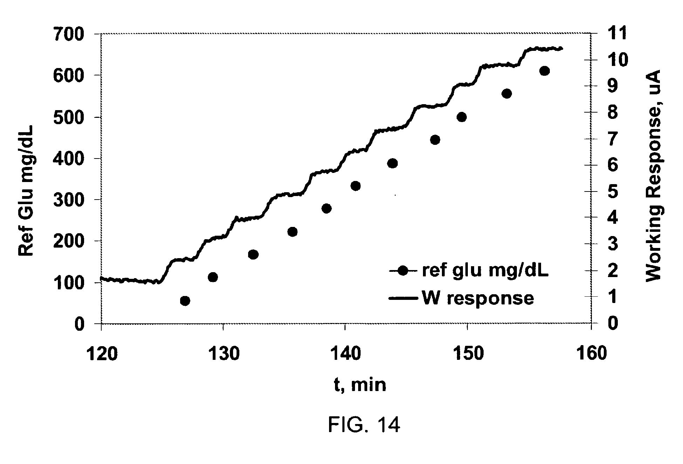

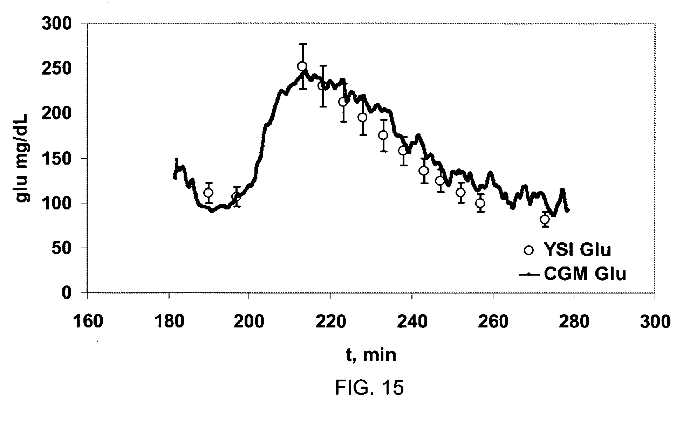

As opposed to continuous in vivo measurements, continuous in vitro measurements of analyte concentration can be observed in discrete steps because there are only very minute changes in the bulk concentration of the analyte due to working electrode consumption. There are no competing physiological reactions consuming the analyte as is the case in vivo. When a diffusion limiting barrier covers the working electrode and there is adequate diffusion control, in vitro electrode response is not affected by mixing or stirring at the outside surface of the diffusion barrier. As a result, discrete stepped responses are easily distinguished and may be used to determine sensor response time. An example of this type of response, from increasing glucose concentrations, is shown in FIG. 14. This is in contrast to continuous in vivo measurements shown in FIG. 15; wherein, the working electrode response from a continuous glucose sensor exhibits a dynamic continuum with no discrete steps. Under these conditions, measurement of sensor response time, in and of itself, is confounded by uptake of glucose by cells and the physiological lag. The current response from a working electrodes takes longer (minutes) than the time for the voltage at the counter electrode to settle (microseconds).

The measurement of dynamic changes in the voltage output to a counter electrode can be accomplished in microseconds such that the in vitro and in vivo response time of a sensor is essentially eliminated, which reduces the overall lag time of an in vivo sensor. For example, the total lag time of in vivo glucose sensors can be on the order of about 20 minutes which includes both physiological and sensor response time. Reducing the sensor response time, reduces the total lag time such that more accurate real-time measurements may be made.

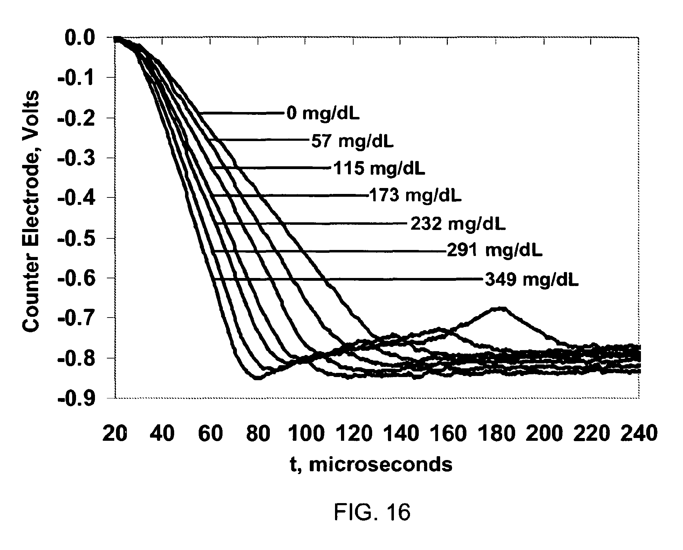

An example of how the output voltage from an op amp to the counter electrode can change when intermittent square wave voltage pulses are applied between a GOx working electrode and a platinum counter electrode, in the presence of glucose, is shown in FIG. 16. As can be seen in FIG. 16, the slope of the voltage output from OP3 (see FIG. 6) to the counter electrode for 0 mg/dL glucose (far right of FIG. 16) is less steep than the slope of the counter electrode voltage for 349 mg/dL glucose (at the far left of FIG. 16). As glucose increases, the slew rate increases and the slope of the output voltage to the counter electrode becomes steeper. This indicates a decrease in the impedance, within the active zone, due to an increase in the number of charge carriers from enzymatic oxidation of glucose by glucose oxidase.

The direct relationship between the op amp voltage output to the counter electrode and glucose concentration is an unexpected finding and represents a novel and rapid analytical tool for determining not only the effects of capacitance and resistance on electrochemical reactions but also yields a method for measuring the concentration of analytes. Rather than waiting minutes for the response of a working electrode to stabilize, a slew rate measurement of the op amp output voltage to a counter electrode takes microseconds. If the faster slew rate measurement of the counter electrode voltage is used to determine glucose concentration and the slower response of the working electrode is also used to determine glucose concentration, the combination of the two methods can result in a redundant rapid system for more accurate measurement of analyte concentrations versus prior art methods.

## EXAMPLE 1

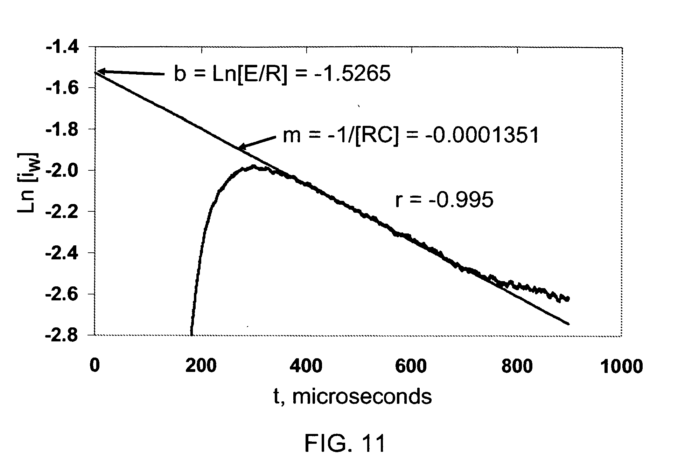

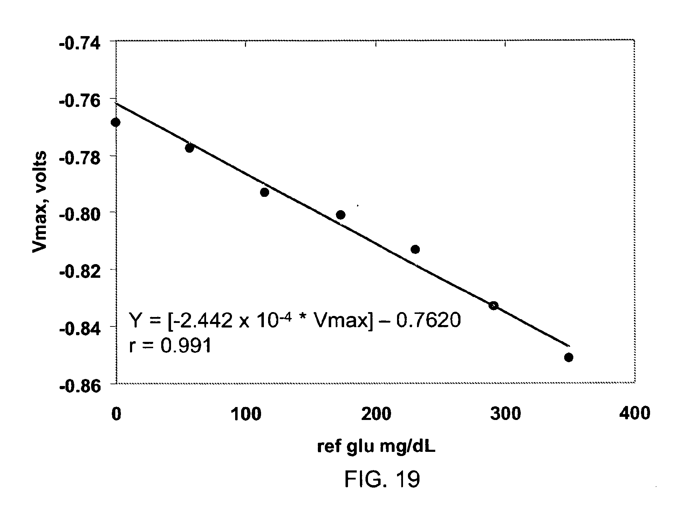

An experiment was carried out in pH 7.4 PBS using the cell configuration shown in FIG. 3. FIG. 10 illustrates the effect of conductance within the active zone as the glucose concentration at the surface of a glucose oxidase working electrode increases. The conductance or reciprocal of resistance on the Y-axis is directly proportional to glucose concentration on the X-axis. The conductance values in FIG. 10 were calculated from resistances obtained from the linear regression of the natural log of the working electrode current versus time as illustrated in FIG. 11 for a single working electrode current vs. time transient. A plot of the natural log ($Ln [i_W]$) of the linear portion of the working electrode current versus time has a slope of -1/RC, and an intercept at zero glucose concentration of $Ln [i_W]_o$ which is also equal to $Ln [E/R]$. The absolute value of the iR drop between the counter and working electrodes (see FIGs. 19, 21, 26a) may be substituted for E and $R_S$ values calculated. The iR drop is the sum of a counter electrode voltage such as, for example, $V_{max}$, $V_{min}$ or $V_f$ or any other counter electrode voltage between $V_{min}$ and $V_f$ and the poise voltage (+0.5000 v). In FIGs. 19, 21 and 26a, the intercepts from linear regression plots of $V_{max}$, $V_{min}$ and $V_f$, versus reference glucose concentration can be used to determine the iR drop of the electrochemical cell. Adding the positive poise voltage and the negative voltage intercepts at zero glucose concentration, the calculated iR drops are -0.2620 v, -0.2107 v and -0.2416 v, respectively. The iR drop calculated from $V_{max}$ may yield a more accurate measurement of the impedance contribution of the electrochemical cell because it is not affected by other processes that occur after $V_{max}$.

When a working electrode such as an amperometric enzyme electrode is implanted in vivo, factors controlling the impedance between the working and counter electrodes and the cell time constant (RC) are more complex than in aqueous buffer. As noted previously, the capacitance ($C_{dl}$) of the double layer at the working electrode surface can be tens of microfarads (1 $\mu$F =$10^{-6}$ F) and the resistance between the working and counter electrodes can be significant, for example, in the Meg Ohm range (M$\Omega$s). Since the electrochemical cell is chemically and electrochemically active, chemical or oxidation/reduction reactions, within the working electrode active zone, can cause changes in the capacitance and resistance between the working and counter electrodes. Increasing the admittance or conductance within the active zone will increase the slew rate of OP3 in FIG. 6 and decrease the slew rate if the admittance or conductance is lower.

There is a time period, prior to the beginning of the working electrode response, wherein electronic components, such as op amps, must settle before the response from the working electrode begins. For example, the "settling time" may be on the order of a few microseconds to perhaps hundreds of microseconds, to achieve the voltage required to maintain a constant voltage between a working and counter electrode. In comparison, the steady-state response of a working electrode may take minutes to achieve a steady state. The response time of op amps, is dependent on the specified nominal slew rate, independent of the impedance of an external load such as an electrochemical cell. Manufacturer specified slew rates can vary from less than one volt per microsecond to hundreds of volts per microsecond. As shown in equation 5, the slew rate is a function of 1/RC as is the glucose concentration within the active zone.

## EXAMPLE 2

In FIG. 12 is shown the results of a single application of a square wave voltage pulse applied between a counter and working electrode of an electrochemical cell incorporating a glucose oxidase working electrode in PH 7.4 PBS (see FIG. 3). The output voltage of OP3 (see FIG. 6) to the counter electrode, is superimposed on the working electrode response (OP1) of the glucose oxidase working electrode. The poise voltage between the working and reference electrode was held constant at +0.50 volts versus an Ag/AgCl reference electrode. In FIG. 12, the voltage output to the counter electrode (OP3) has a negative slope; and the positive working electrode response (W) does not begin until the voltage output of the counter electrode, OP3, has settled which, in this case, is about 200 microseconds.

Referring to FIG. 12, in order to maintain a +0.50 volt difference between the reference and working electrode, the output voltage from OP3, had to drop to about -0.90 volts to overcome the impedance contributed by the electrochemical cell (iR drop of about -0.40 V). In the absence of added impedance from the electrochemical cell, the final voltage from the counter electrode would likely be closer to -0.50 volts rather than -0.90 volts.

## EXAMPLE 3

The cell configuration shown in FIG. 3 was used and a potentiostat (see FIG. 6) having a waveform generator interfaced to a lap-top computer was used to apply periodic square wave voltage pulses between the working and counter electrodes of an electrochemical cell.

A 10.0 mL glass cylindrical cell (BioAnalytical Systems Inc) was filled with 5.0 mL of pH 7.4 phosphate buffered saline (PBS). The counter electrode consisted of a coiled length of platinum wire and the reference electrode was a silver wire having a silver chloride coating. The working electrode consisted of a 0.35 mm diameter platinum-iridium (80: 20) wire dip coated with a solution of 5% gelatin containing 3% GOx dissolved in pH 7.4 PBS. The enzyme coated wire was cured at 70°C in a mechanical convection oven for 30 minutes. This was followed by dip coating the enzyme coated wire once in 5% aqueous glutaraldehyde, followed by curing at 70°C for 30 minutes. The cured, crosslinked enzyme coated wire was then dip coated in a 3% solution of polyurethane dissolved in tetrahydrofuran (THF), followed by oven curing at 70°C for 30-60 minutes. The assembled electrode was allowed to cure at room temperature for 12-24 hours before use.

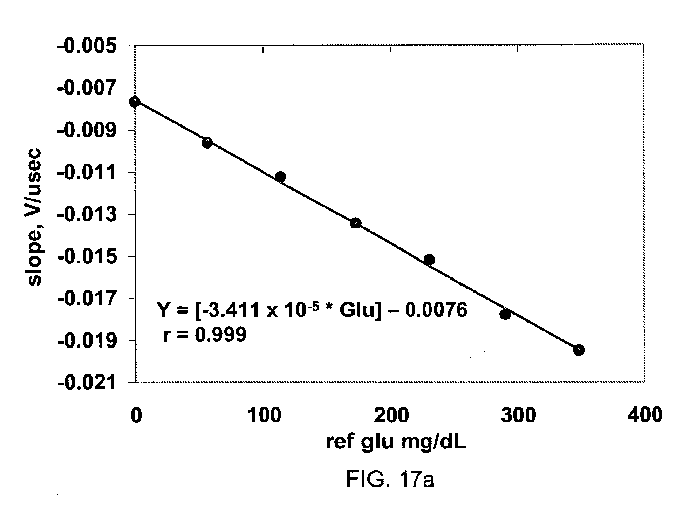

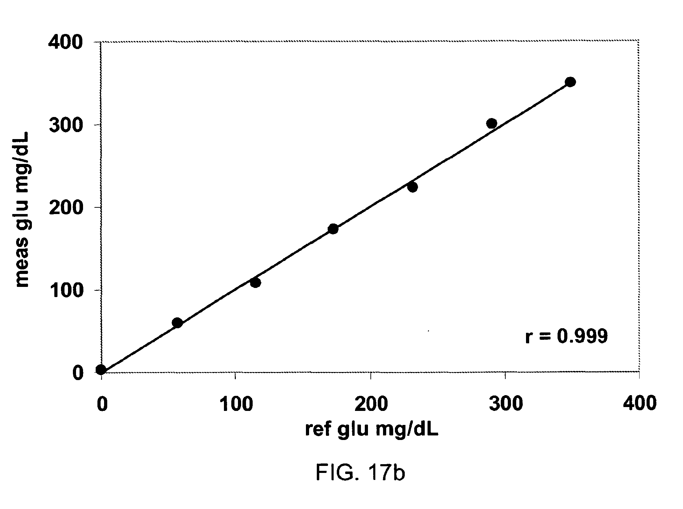

The slope of the linear, falling portion of the output voltage to the counter electrodes was determined for each glucose concentration in FIG. 16. Linear regression of the rate of change of the counter electrode voltage versus time, for each of the glucose concentrations shown in FIG. 16, yielded a slope and intercept for each glucose concentration. The slope [dV/dt] for each glucose concentration on the Y-axis was linear regressed against reference glucose concentrations on the X-axis as shown in FIG. 17a. In FIG. 17a, the slew rate is linear versus reference glucose concentration (r = 0.999). The slope of the graph in FIG. 17a is negative (-3.411 x $10^{-5}$ volts/usec) because the counter electrode behaves as the negative (cathode) electrode. The slope of the output voltage to the counter is actually increasing with increasing glucose concentration. If reduction was occurring at the working electrode the sign of the counter electrode slew rate plot would be positive vs. analyte concentration. Using the slope and intercept data from FIG. 17a, glucose concentrations for each slew rate value were calculated. The calculated glucose concentrations on the Y-axis were linear regressed against reference glucose concentration on the X-axis (r = 0.999) as shown in FIG. 17b.

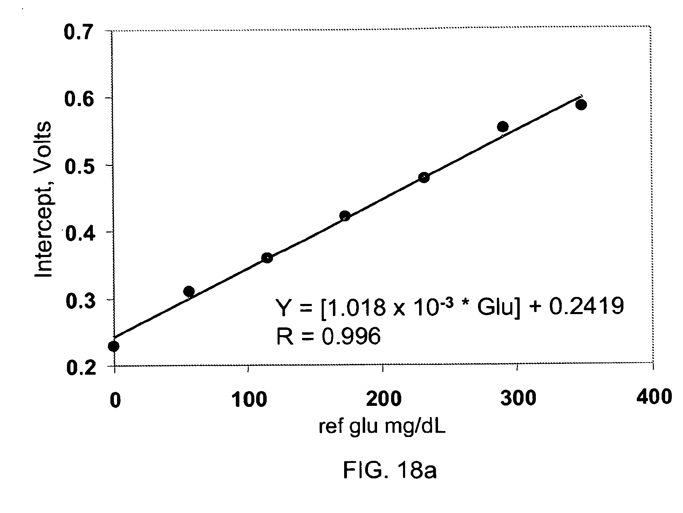

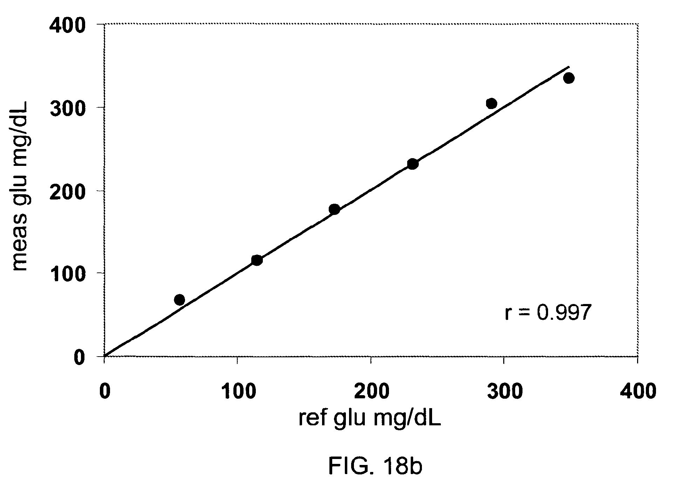

In the same manner as for the slopes above, a plot of the intercept from each linear regression of the rate of change of the counter electrode voltage versus reference glucose concentration also yields a linear response to glucose concentration as shown in FIG. 18a. Using the slope and intercept from FIG. 18a, measured glucose concentrations, for each of the intercept values, are graphed on the Y-axis and linear regressed against reference glucose concentrations on the X-axis as shown in FIG. 18b (r = 0.997).

For each of the glucose concentrations shown in FIG. 16, the corresponding values (see FIG. 1) of $V_{max}$, $tV_{max}$, $1/tV_{max}$, $V_{min}$, $1/tV_{min}$ and $V_f$ were calculated. FIG. 19 shows a linear regression graph of $V_{max}$ on the Y-axis versus reference glucose concentration on the X-axis. As shown in FIG. 19, the slope is equal to $-2.442 \times 10^{-4}$ volts per mg/dL and the y-intercept is -0.7620 volts at zero glucose concentration with r = 0.991.

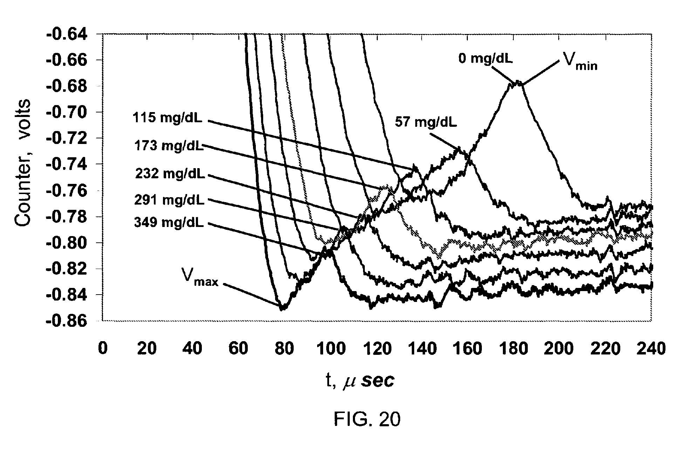

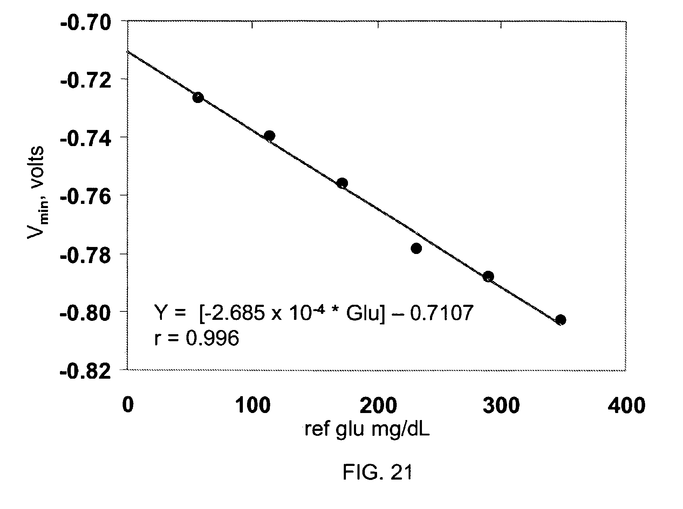

FIG. 20 shows an expanded view of the data in FIG. 16. FIG. 20 shows glucose concentrations associated with each $V_{min}$ peak value. A linear regression plot of $V_{min}$ on the Y-axis versus reference glucose concentration on the X-axis is shown in FIG. 21 with a correlation coefficient of 0.996. In the case of $V_{min}$, the iR drop between the working and counter electrodes is equal to: -0.7107 v + 0.5000 v = -0.2107 v. This iR drop is less than that calculated from $V_{max}$ in FIG 19.

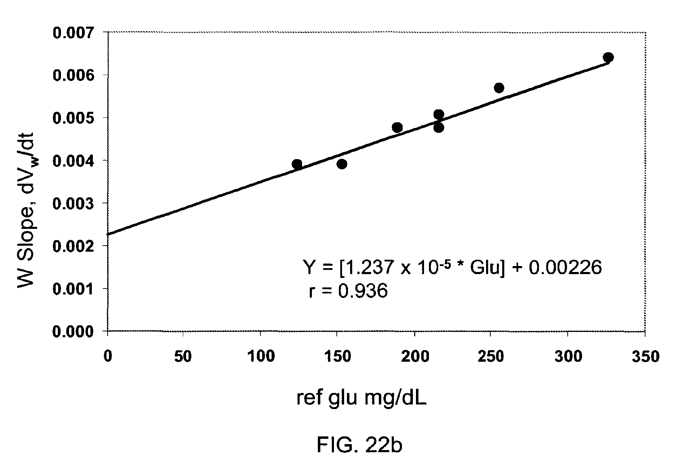

The lower iR drop at $[V_c]_{min}$, where the concentration of charge is at a maximum, is mirrored by the working electrode voltage minimum in FIG 22a, wherein the working electrode voltage and counter electrode voltage are graphed versus time. There is a distinct change in the working electrode voltage corresponding to the counter electrode voltage at $[V_c]_{min}$. It appears that the increase and decrease in the counter voltage on either side of $[V_c]_{min}$ is a result of increasing charge prior to $[V_c]_{min}$, followed by the decreasing charge after $[V_c]_{min}$. Because $[V_c]_{min}$ is linear with glucose concentration, it appears that the charging and discharging phenomenon is not totally due to double layer charging ($C_{dl}$), there are other contributions possible from chemical and/or electrochemical reactions of the analyte. This is evident by the magnitude of the working electrode voltage of about +0.46 volts between voltage pulses as shown on the right Y-axis of FIG. 22a. This residual voltage at the working electrode is sufficient to cause some oxidation of hydrogen peroxide in the off-time between pulses. This is an unexpected benefit, because a portion of hydrogen peroxide is consumed between pulses and this lessens the effect of excess hydrogen peroxide on GOx degradation and/or denaturation. FIG. 22b shows a linear regression plot of the rate of change of the initial rise in working electrode voltage $[dV_w/dt]$ on the Y-axis versus reference glucose concentration on the X-axis (r = 0.936).

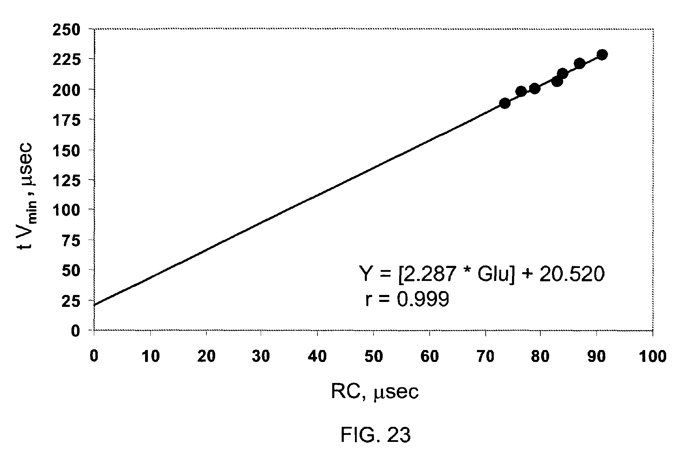

FIG. 23 shows the relationship of $tV_{min}$ in microseconds to the RC time constant derived for equation 5. The measured values of $tV_{min}$ on the Y-axis were linear regressed versus RC time constant values on the Y-axis, r = 0.999, indicating the direct relationship between time and the RC time constant.

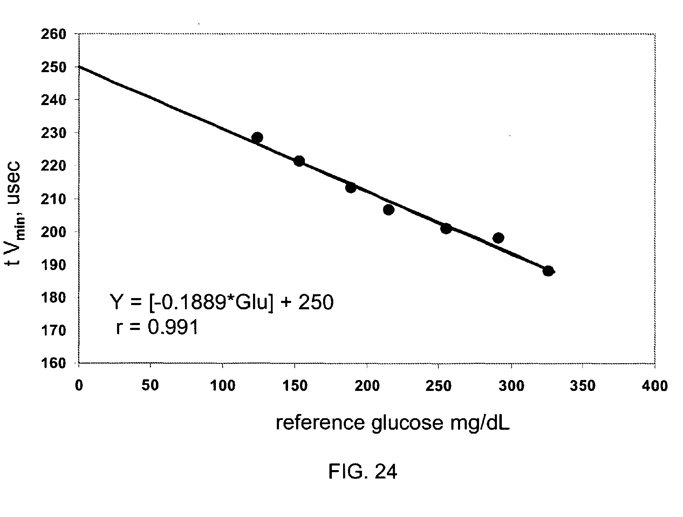

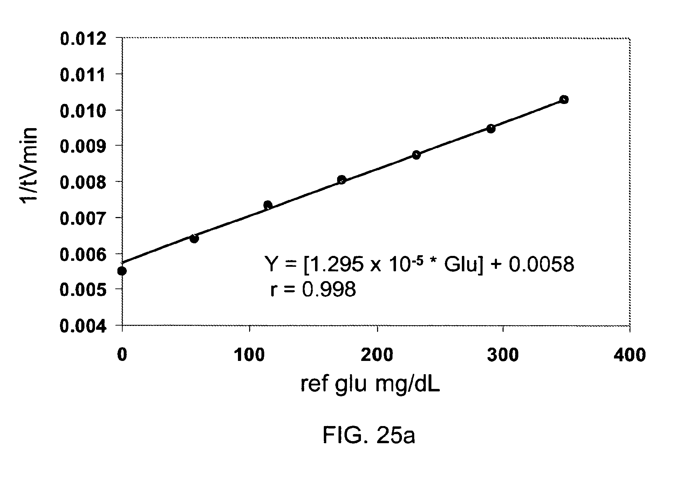

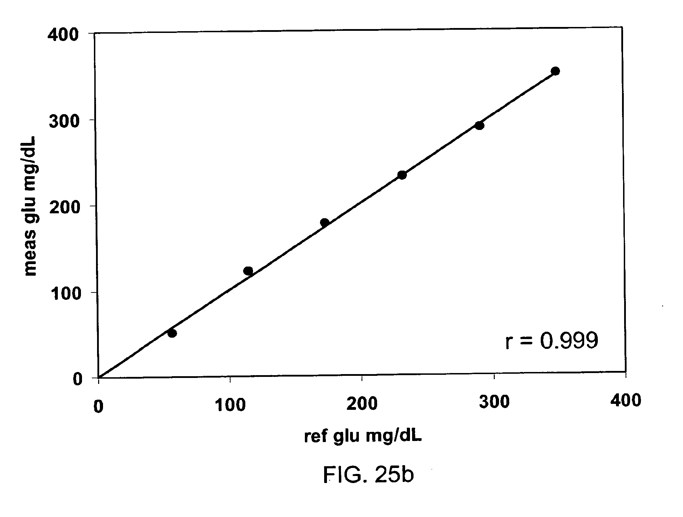

FIG. 24 shows a linear regression graph of the time ($tV_{min}$) at each value of $V_{min}$ on the Y-axis, plotted versus reference glucose concentration on the X-axis, r = 0.991. The reciprocal values of the t $V_{min}$ values in FIG. 24, $1/tV_{min}$, on the Y-axis were linear regressed versus reference glucose concentration on the X-axis as shown in FIG. 25a. The slope and intercept from the linear regression data in FIG. 25a were used to calculate glucose concentrations from each $1/tV_{min}$ value. A linear regression correlation plot of calculated glucose concentration versus reference glucose concentrations is shown in FIG. 25b. The reciprocal values of time ($1/t_{Vmin}$, r = 0.998) provide a better fit for the data versus that shown in FIG 24 for $tV_{min}$ (r = 0.991).

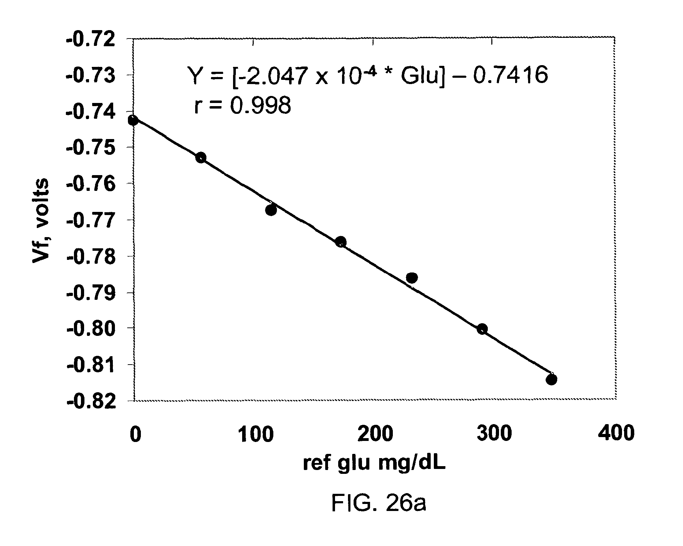

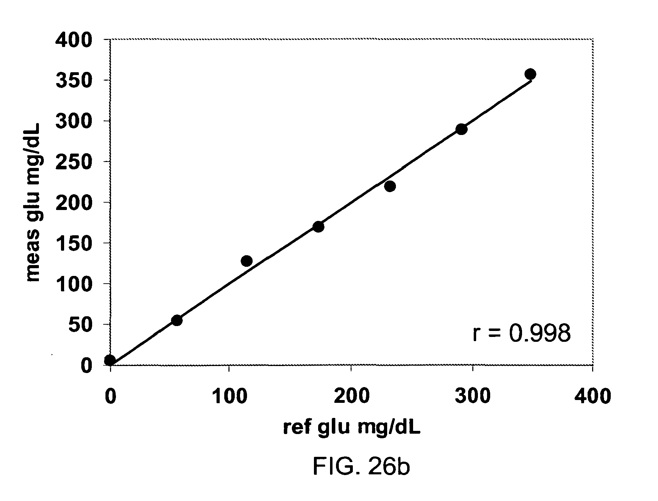

A linear regression plot of $V_f$ on the Y-axis, determined from the average voltage over the last 50 $\mu$sec of measurement, versus reference glucose concentration on the X-axis is shown in FIG. 26a, the slope and intercept were used to calculate glucose concentrations for each $V_f$ voltage and linear regressed against reference glucose concentration as shown in FIG. 26b, r = 0.998. The iR drop at $V_f$ is equal to: -0.7416 v + 0.0005 v = -0.2416 v. This iR drop value is greater than $V_{min}$ but less than for $V_{max}$.

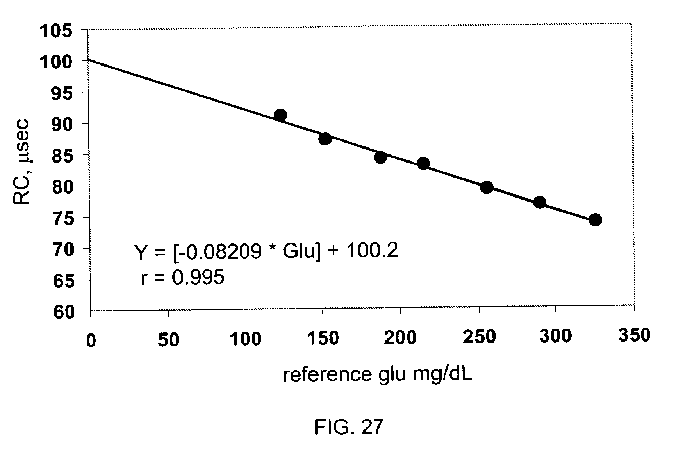

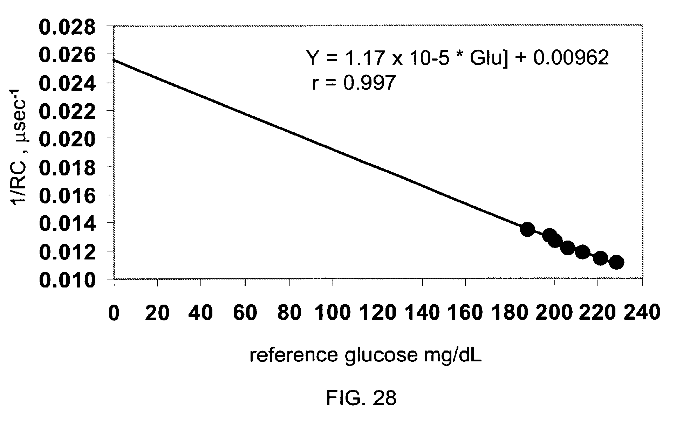

By rearranging equation 5, RC or 1/RC values may be calculated by substituting $V_{min}$ for E and dividing the measured slew rate by $V_{min}$ (or $V_{max}$ or $V_f$). A linear regression graph of RC values on the Y-axis versus glucose concentration on the X-axis is shown in FIG. 27, r = 0.995. This graph shows that as glucose increases, the RC time constant decreases from about 100 microseconds at zero glucose concentration to approximately 50 microseconds at 325 mg/dL glucose in line with how the slope of the counter electrode voltage changes versus glucose concentration. In addition, a linear regression plot of 1/RC on the Y-axis versus reference glucose concentration on the X-axis yields a linear relationship with glucose concentration as shown in FIG. 28 (r = 0.997).

## EXAMPLE 4

Instead of using aqueous buffer as in EXAMPLE 3, an experiment was conducted in bovine citrated plasma, using the cell configuration in FIG. 3. The purpose of this experiment was to study the effect of stimulated fibrin formation on the outside surface of a diffusion limiting barrier over an enzyme electrode. The set-up procedure in EXAMPLE 3 was followed, but the cell was filled with 5 mL of citrated bovine plasma instead of pH 7.4 PBS. The same working electrode preparation method was used as described in EXAMPLE 3. The background concentration of glucose in the citrated plasma was measured with a Yellow Springs Instruments Model 2300 Glucose Analyzer and found to be 124 mg/dL. Continuous, square wave voltage pulses, with an on-time ($\tau_1$) of 0.5 seconds and an off-time ($\tau_2$) of 4.5 seconds, were applied between the counter and working electrodes. The voltage difference between the working electrode and a silver-silver chloride reference electrode was maintained at +0.50 volts. A data sampling rate of 5.0 MHz was used to yield one data point every 0.2 $\mu$sec. Only data obtained within the first 1,000 $\mu$sec (1 millisecond) of each voltage pulse was saved in memory and used for subsequent analyses.

After an equilibration period of approximately 30-60 minutes, 5$\mu$L additions of a 50% aqueous solution of $\beta$-D glucose were made incrementally. The time between additions of glucose can vary, but it is important to allow the output from the working electrode to reach a steady-state between additions (e.g. 5 to 10 minutes). In addition to the background glucose concentration of 124 mg/dL, three glucose additions were made to provide four glucose concentrations (i.e. initial background concentration, plus three 5 $\mu$L aliquots of glucose) for determination of the slew rate of the op amp output voltage to the counter electrode versus each glucose concentration. Following the third glucose addition, a small amount of thromboplastin and calcium was added to stimulate localized clotting on the diffusion limiting membrane surface over the working electrode without clotting the entire solution of citrated plasma. Thromboplastin with calcium was titrated so that fibrin formation was localized on the working electrode diffusion limiting barrier, where fibrinogen had adsorbed, with no clotting of the citrated plasma solution. Following an equilibration period of 30 minutes, three more 5 $\mu$L additions of glucose were made to bring the final concentration of glucose to 326 mg/dL.

The rate of change of the op amp voltage output to the counter electrode, slew rate = $[dV/dt]_{max}$ or slope (V/$\mu$sec) (see FIG. 17a) on the Y-axis was determined for each reference glucose concentration on the X-axis (7 including the starting glucose concentration of 124 mg/dL in the citrated plasma and 6 further additions of glucose); however, only the first four slope values were linear regressed against initial glucose concentration and the first 3 glucose additions (calibration set) as shown in FIG. 29. Note that in FIG. 29, the last three slew rates for 256, 291 and 326 mg/dL, respectively (not included in the linear regression calibration set) are above the linear regression line indicating lower conductance within the active zone of the working electrode due to decreasing mass transfer of glucose across the diffusion limiting barrier on the working electrode.

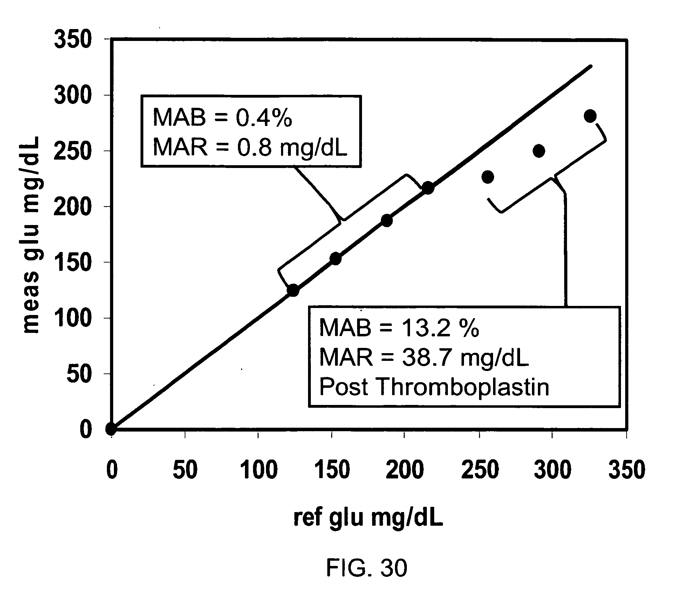

After addition of thromboplastin and calcium, the slope vs. glucose concentration in FIG. 29 decreased (last three points, less negative). Using the slope and intercept data obtained using the calibration set from FIG. 29, glucose concentrations were calculated on all data before and after thromboplastin addition. The error caused by fibrin formation on the sensor surface, is illustrated in FIG. 30 where the calculated glucose concentrations after thromboplastin addition exhibited a mean absolute bias (MAB) of 13.2% and a mean absolute residual (MAR) of 38.7 mg/dL. In contrast, the data before thromboplastin addition had a corresponding MAB of 0.4% and MAR or 0.8 mg/dL, respectively. Fibrin formation, on the outside surface of the diffusion limiting membrane, reduces the surface area for glucose mass transport so that less mass of glucose enters the active zone and thus the magnitude of charge formation, from the enzymatic oxidation (chemical) of glucose and subsequent electrochemical oxidation of hydrogen peroxide, within the active zone, is reduced as is the rate of change of the counter electrode voltage.

BIOFOULING CORRECTION

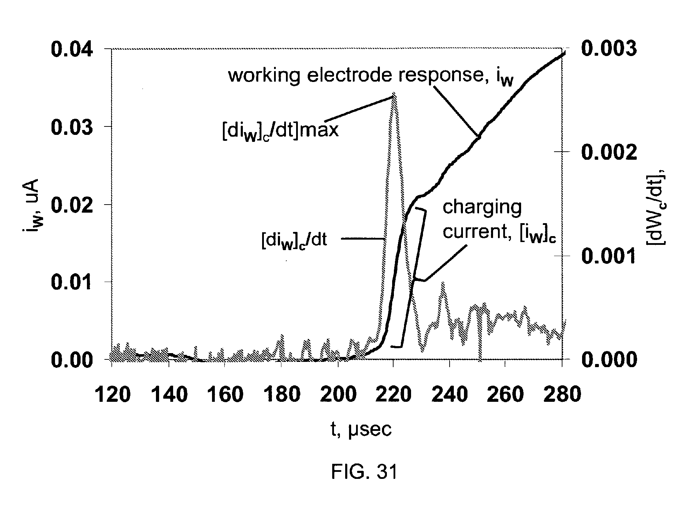

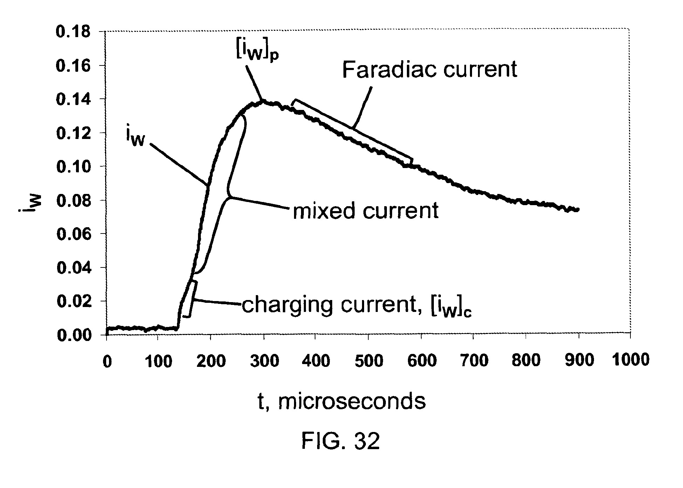

A method to correct for the error caused by biofouling is illustrated in FIG. 31 which shows a plot of the working electrode response current ($i_W$) (black solid trace) versus the first derivative (gray solid trace) with respect to time of the initial charging current $[d_{Wc}/dt]$ as delineated by the bracket (labeled charging current) enclosing the initial part of the rising working electrode response, the maximum of the first derivative of the charging current is denoted as $[di_{Wc}/dt]_{max}$ which is also expressed as $[i_{Wc}]'_{max}$. In FIG. 32, the working electrode current response shows a peak current denoted as $I_p$. The charging current response illustrated in FIG. 32, has a steeper slope than the current response after the charging current. As the working electrode current increases beyond the charging current response, the slope decreases because the current response is due to slower processes than the initial charging current response, this results in the inflection point at $[di_{Wc}/dt]$ max. Biofouling and other aspects of biosensors are disclosed in U.S. Patent Application Publication No. 2007/0299617 to Willis, which is incorporated herein in its entirety.

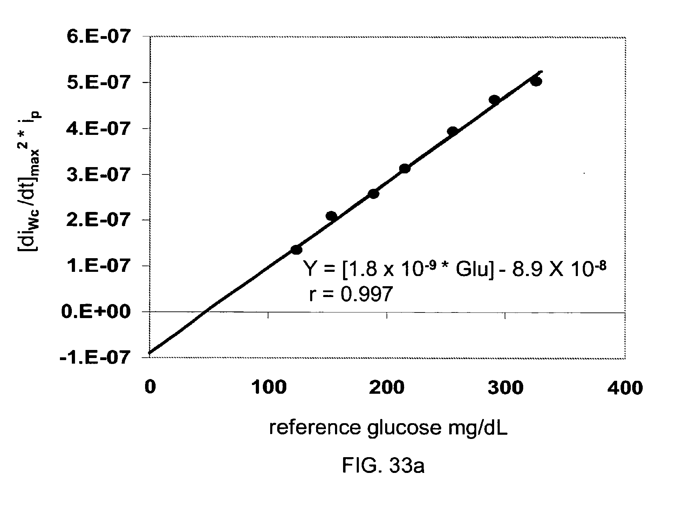

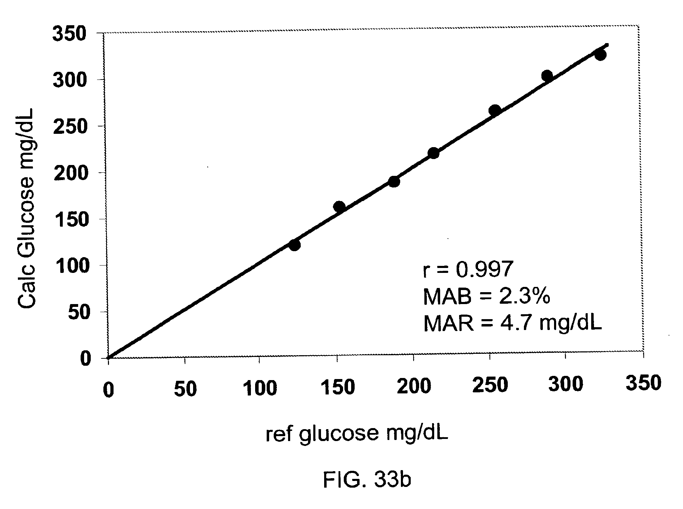

Since the data plotted in FIGs. 29 & 30 showed a decrease in the slew rate with biofouling, various functions were examined to determine whether there was a function or set of functions that were independent of biofouling at any analyte concentration. One such function is the product of the maximum rate of change of the charging current squared $\{[dWc/dt]_{max}\}^2$ times the working electrode peak current response ($I_p$). As shown in FIG. 33a a linear regression plot of $\{[di_{Wc}/dt]_{max}\}^2 * I_p$ on the Y-axis, determined from the glucose concentrations shown in FIG 29, versus reference glucose concentration on the X-axis is linear with glucose concentration (r = 0.997), even in the presence of fibrin formation on the biosensor membrane surface. A linear regression correlation plot of measured glucose on the Y- axis versus reference glucose concentration on the X- axis is shown in FIG. 33b. In this instance, there is no fall- off in glucose response in the presence of thromboplastin and calcium as shown in FIG. 29. In FIG. 33b, the mean absolute bias over the entire range of glucose concentration was 2.3% and the mean absolute residual was 4.7 mg/dL.

Although the foregoing invention has been described in terms of certain embodiments, numerous and varied other embodiments can be readily devised by those skilled in the art. Accordingly, the descriptions of the present invention should be taken as illustrating rather than limiting the invention as claimed.

## Claims

1. A method for determining a concentration of at least one analyte, the method comprising:

   providing an electrochemical cell comprising a working electrode coated with a protein layer and a diffusion limiting barrier covering the protein layer, and a counter electrode; a voltage source which provides a voltage between the working electrode and the counter electrode; and a computing system which measures a voltage output to the counter electrode;
   positioning the working electrode and the counter electrode in an electrically conductive medium;
   applying the voltage from the voltage source to the working electrode and the counter electrode;
   measuring the voltage output applied to the counter electrode prior to the working electrode response; and
   determining the analyte concentration based on the measured voltage output applied to the counter electrode.

2. The method of claim 1, wherein determining the analyte concentration is based on a rate of change of the voltage applied to the counter electrode, $V_c$, $V_{min}$, $tV_{min}$, $1/tV_{min}$, $V_{max}$, $tV_{max}$, $1/tV_{max}$, RC, 1/RC Or $V_f$.

3. The method of claim 1, wherein measuring the voltage output applied to the counter electrode prior to the working electrode response is within less than about 200 microseconds.

4. The method of claim 1, wherein applying the voltage from the voltage source comprises applying a voltage waveform.

5. The method of claim 1, wherein the measuring further comprises measuring the rate of change of the voltage output applied to the counter electrode between at least two voltage time points yielding a value proportional to analyte concentration at the working electrode.

6. The method of claim 1, wherein the voltage source is a potentiostat.

7. The method of claim 4, wherein applying the voltage waveform causes the voltage output applied to the counter electrode form an operational amplifier of the voltage source to slew each time the voltage waveform is applied.

8. The method of claim 1, wherein the counter electrode is in contact with the diffusion limiting barrier.

9. The method of claim 1, further comprising providing a reference electrode.

10. The method of claim 1, wherein the diffusion limiting barrier comprises a polymeric material.

11. The method of claim 10, wherein the polymeric material comprises a polyurethane.

## Patentansprüche

1. Verfahren zum Bestimmen einer Konzentration von wenigstens einem Analyten, wobei das Verfahren umfaßt:

   - ein Bereitstellen einer elektrochemischen Zelle, die umfaßt: eine Arbeitselektrode, die beschichtet ist mit einer Protein-Schicht und einer eine Diffusion beschränkenden Barriere, die die Protein-Schicht bedeckt, und eine Gegenelektrode; eine Spannungsquelle, die eine Spannung zwischen der Arbeitselektrode und der Gegenelektrode liefert; und eine Computeranlage, die eine Spannungsausgabe an die Gegenelektrode mißt;
   - ein Positionieren der Arbeitselektrode und der Gegenelektrode in einem elektrisch leitenden Medium;
   - ein Anlegen der Spannung von der Spannungsquelle an die Arbeitselektrode und die Gegenelektrode;

- ein Messen der an die Gegenelektrode angelegten Spannungsausgabe vor der Reaktion der Arbeitselektrode; und

- ein Bestimmen der Analyt-Konzentration auf der Basis der gemessenen, an die Gegenelektrode angelegten Spannungsausgabe.

2.  Verfahren nach Anspruch 1, worin ein Bestimmen der Analyt-Konzentration basiert wird auf einer Änderungsrate der Spannung, die an die Gegenelektrode angelegt wurde, $V_C$, $V_{min}$, $tV_{min}$, $1/tV_{min}$, $V_{max}$, $tV_{max}$, $1/tV_{max}$, RC, 1/RC oder $V_f$.

3.  Verfahren nach Anspruch 1, worin ein Messen der an die Gegenelektrode angelegten Spannungsausgabe vor der Reaktion der Arbeitselektrode innerhalb von weniger als etwa 200 Mikrosekunden erfolgt.

4.  Verfahren nach Anspruch 1, worin ein Anlegen der Spannung von der Spannungsquelle ein Anlegen einer Spannungs-Wellenform umfaßt.

5.  Verfahren nach Anspruch 1, worin das Messen weiter ein Messen der Änderungsrate der an die Gegenelektrode angelegten Spannungsausgabe zwischen wenigstens zwei Spannungs-Zeit-Punkten umfaßt, was zu einem Wert proportional zur Analyt-Konzentration an der Arbeitselektrode führt.

6.  Verfahren nach Anspruch 1, worin die Spannungsquelle ein Potentiostat ist.

7.  Verfahren nach Anspruch 4, worin ein Anlegen der Spannungs-Wellenform dazu führt, daß sich die an die Gegenelektrode angelegte Spannungsausgabe von einem betriebsbedingten Verstärker der Spannungsquelle jedes Mal umkehrt, wenn die Spannungs-Wellenform angelegt wird.

8.  Verfahren nach Anspruch 1, worin die Gegenelektrode in Kontakt mit der eine Diffusion begrenzenden Barriere ist.

9.  Verfahren nach Anspruch 1, weiter umfassend das Bereitstellen einer Referenzelektrode.

10. Verfahren nach Anspruch 1, worin die eine Diffusion begrenzende Barriere ein Polymermaterial umfaßt.

11. Verfahren nach Anspruch 10, worin das Polymermaterial ein Polyurethan umfaßt.


**Revendications**

1.  Procédé de détermination d'une concentration d'au moins un analyte, le procédé comprenant les étapes consistant à :

    fournir une cellule électrochimique comprenant une électrode de travail revêtue avec une couche de protéine et une barrière limitant la diffusion couvrant la couche de protéine, et une contre-électrode ; une source de tension qui fournit une tension entre l'électrode de travail et la contre-électrode ; et un système informatique qui mesure une tension fournie en sortie à la contre-électrode ;
    positionner l'électrode de travail et la contre-électrode dans un milieu électriquement conducteur ;
    appliquer la tension de la source de tension à l'électrode de travail et à la contre-électrode ;
    mesurer la sortie de tension appliquée à la contre-électrode avant la réponse de l'électrode de travail ; et
    déterminer la concentration en analyte en se basant sur la sortie de tension mesurée appliquée à la contre-électrode.

2.  Procédé selon la revendication 1, dans lequel la détermination de la concentration en analyte est basée sur un taux de changement de la tension appliquée à la contre-électrode, $V_c$, $V_{min}$, $tV_{min}$, $1/tV_{min}$, $V_{max}$, $tV_{max}$, $1/tV_{max}$, RC, 1/RC ou $V_f$.

3.  Procédé selon la revendication 1, dans lequel la mesure de la sortie de tension appliquée à la contre-électrode avant la réponse de l'électrode de travail se fait en moins d'environ 200 microsecondes.

4.  Procédé selon la revendication 1, dans lequel l'application de la tension à partir de la source de tension comprend l'application d'une forme d'onde de tension.

**EP 2 416 893 B1**

5. Procédé selon la revendication 1, dans lequel la mesure comprend en outre la mesure du taux de changement de la sortie de tension appliquée à la contre-électrode entre au moins deux instants de tension donnant une valeur proportionnelle à la concentration en analyte au niveau de l'électrode de travail.

6. Procédé selon la revendication 1, dans lequel la source de tension est un potentiostat.

7. Procédé selon la revendication 4, dans lequel l'application de la forme d'onde de tension amène la sortie de tension appliquée à la contre-électrode provenant d'un amplificateur opérationnel de la source de la tension à effectuer un balayage chaque fois que la forme d'onde de tension est appliquée.

8. Procédé selon la revendication 1, dans lequel la contre-électrode est en contact avec la barrière limitant la diffusion.

9. Procédé selon la revendication 1, comprenant en outre l'étape consistant à fournir une électrode de référence.

10. Procédé selon la revendication 1, dans lequel la barrière limitant la diffusion comprend un matériau polymérique.

11. Procédé selon la revendication 10, dans lequel le matériau polymérique comprend un poly(uréthane).

23

FIG. 1

Analyte

Conductive Medium

C    W    Ref

Potentiostat

$\Delta E_{wr}$

Glass Cell

FIG. 2

FIG. 3

FIG. 4

FIG. 5

FIG. 6

FIG. 7a

FIG. 7b

FIG 8a

FIG. 8b

FIG. 9a

FIG. 9b

FIG. 10

FIG. 11

counter electrode voltage

working electrode current

counter $V_c$, volts (e.g., op3 **Fig. 6**)

working $i_w$, uA (e.g. op1 **FIG. 6**)

-0.9 v

settling time

t, microseconds

FIG. 12

FIG. 13

FIG. 14

FIG. 15

FIG. 16

FIG. 17a

FIG. 17b

$$Y = [1.018 \times 10^{-3} * Glu] + 0.2419$$
$$R = 0.996$$

FIG. 18a

FIG. 18b

$$Y = [-2.442 \times 10^{-4} * Vmax] - 0.7620$$
$$r = 0.991$$

FIG. 19

FIG. 20

FIG. 21

FIG. 22a

$$Y = [1.237 \times 10^{-5} * Glu] + 0.00226$$
$$r = 0.936$$

FIG. 22b

FIG. 23

FIG. 24

Y = [1.295 x 10$^{-5}$ * Glu] + 0.0058
r = 0.998

FIG. 25a

FIG. 25b

$$Y = [-2.047 \times 10^{-4} * Glu] - 0.7416$$
$$r = 0.998$$

FIG. 26a

FIG. 26b

FIG. 27

FIG. 28

FIG. 29

FIG. 30

FIG. 31

FIG. 32

FIG. 33a

FIG. 33b

1. | In vitro or in vivo electrochemical cell in conductive fluid |

2. | activate cell: begin application of voltage between counter & working |

3. | record counter and working electrode response(s) |

4a. | calculate voltage function(s) of counter electrode ($V_c$) |

4b. | calculate function(s) of working electrode $i_w$, $V_w$ |

5. | Filter Data |

6a. | calculate analyte concentration $f(V_c)$ |

6b. | calculate analyte concentration $f(i_w)$ |

FIG. 34

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070299617 A, Willis